(19)

Europäisches
Patentamt
European
Patent Office
Office européen
des brevets

(11) **EP 2 620 506 A1**

(12) **EUROPEAN PATENT APPLICATION**

(43) Date of publication:
**31.07.2013 Bulletin 2013/31**

(51) Int Cl.:
***C12P 19/00*** *(2006.01)*    ***C12P 19/04*** *(2006.01)*
***C12P 19/16*** *(2006.01)*    ***C12P 19/20*** *(2006.01)*

(21) Application number: **12152502.6**

(22) Date of filing: **25.01.2012**

---

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Arla Foods Amba
8260 Viby J (DK)**

(72) Inventors:
• **Bertelsen, Hans
6920 Videbæk (DK)**

• **Wejse, Peter Langborg
8200 Aarhus N (DK)**
• **Busch, Jon Weis
7100 Vejle (DK)**

(74) Representative: **Østergaard, Steen
Guardian IP Consulting I/S
Diplomvej, Building 381
2800 Kgs. Lyngby (DK)**

---

(54) **Method of producing a galacto-oligosaccharide-containing composition**

(57)    The present invention relates to a method of producing compositions containing galacto-oligosaccharides as well as to galacto-oligosaccharide-containing compositions as such.

EP 2 620 506 A1

**Description**

**FIELD OF THE INVENTION**

The present invention relates to a galacto-oligosaccharide-containing composition as well as an efficient method of producing it.

**BACKGROUND**

Human breast milk is known to contain a number of different oligosaccharides which are ascribed some of the beneficial health effects of breast feeding infants (Kunz *et al.* (2000)). For example, some oligosaccharides, such as FOS, GOS or inulin, are so-called prebiotics, which means that they promote the beneficial bacteria of the gastrointestinal system and disfavour the harmful bacteria. Oligosaccharides are, due to their health promoting effects, frequently used in functional food products, such as infant formulas and clinical nutrition.

There are several approaches to the production of oligosaccharides. One approach is based on isolating oligosaccharides from naturally occurring sources. Fructose-oligosaccharide (FOS) and inulin are for example found naturally in Jerusalem artichoke, burdock, chicory, leeks, onions and asparagus and may be isolated from these crops. Preparation of inulin from chicory roots is e.g. described in Frank (2002). This approach to the production of oligosaccharides is limited by the availability of suitable crops and may be impossible to implement for more complex oligosaccharides.

Another approach is based on enzymatic synthesis in which enzymes catalyse the synthesis of the oligosaccharides. Yun (1996) describes the enzymatic production of fructo-oligosaccharides using enzymes having fructosyltransferase activity and using sucrose as substrate for the enzyme. Another example of enzymatic synthesis is described in WO 01/90,317 A2 which discloses a method of producing galacto-oligosaccharides (GOS) of the formula Gal-Gal-Glc using a special beta-galactosidase enzyme and lactose as substrate.

**SUMMARY OF THE INVENTION**

An object of the present invention is to provide improved methods of producing galacto-oligosaccharides, and particularly galacto-oligosaccharides having a different reducing end than the galactosyl donor used during the production.

The present inventors have found that, surprisingly, leaving groups released from the donor during synthesis of galacto-oligosaccharides act as competing galactosyl acceptors and reduces the yield of the above-mentioned galacto-oligosaccharides. This is particularly surprising as initial trials performed by the inventors have indicated that leaving groups, and particularly glucose, are poor galactosyl acceptors. The present inventors have furthermore discovered that the yield of the above-mentioned galacto-oligosaccharides (having a different reducing end than the galactosyl donor) may be increased by removing released leaving groups from the reaction mixture during the incubation of galactosyl donor, galactosyl acceptor and beta-galactosidase enzyme.

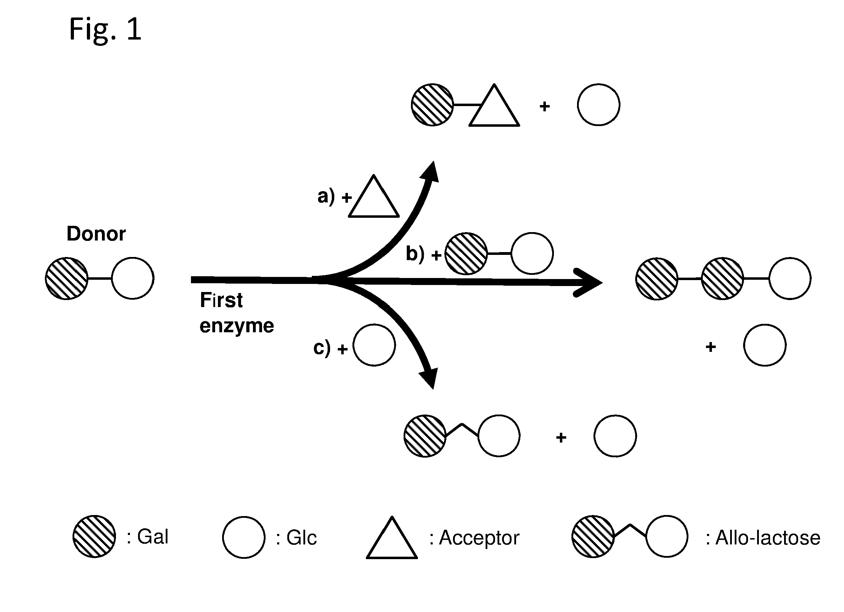

Figures 1, 2 and 3 explain this in further detail.

Figure 1 is a schematic depiction of the main types of reactions that takes place during transgalactosylation. Reaction a) is the desired reaction and involves the transferral of a galactosyl group from the donor (lactose in this example) to the acceptor (e.g. fucose). The products of this reaction is a free leaving group (glucose in this example) and a small oligosaccharide (e.g. Gal-Fuc) having a different reducing end than the galactosyl donor.

Reaction b) is an undesired side reaction which leads to so-called self-galactosylation of the donor, e.g. further galactosylated lactose if the donor is lactose. This by-product is difficult and expensive to remove from the oligosaccharide product. The present inventors have discovered that the level of self-galactosylation can be reduced significantly by using a first enzyme having a high transgalactosylation efficiency (a low T-value) in combination with a relatively low concentration of galactosyl donor.

Reaction c) of Figure 1 is another undesired side reaction which the present inventors have recently discovered. The present inventors have observed surprisingly high levels of allo-lactose in galacto-oligosaccharide compositions and have come to the conclusion that free leaving groups, and particularly, glucose also act as acceptor if its concentration is sufficiently high.

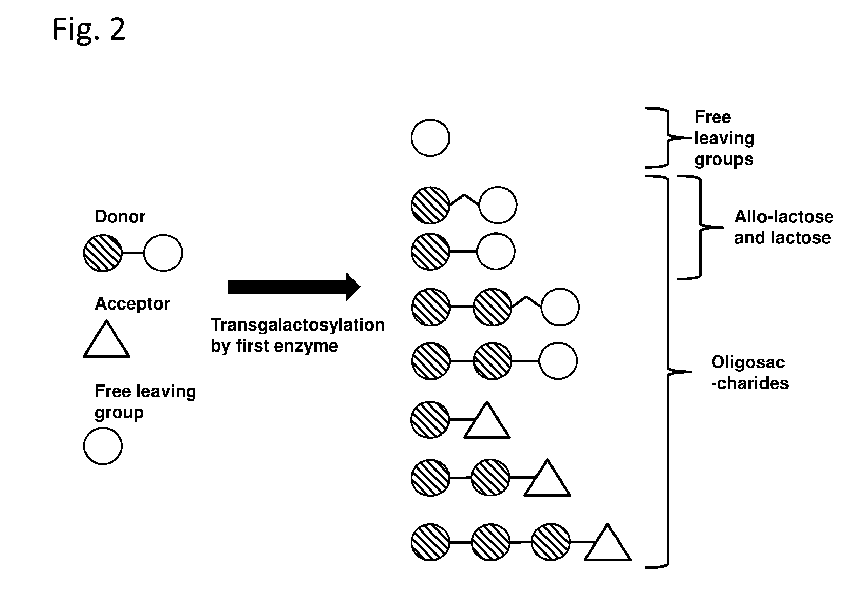

Figure 2 is a schematic illustration of some of the molecular species present in the galacto-oligosaccharide compositions when the free leaving groups are not removed during incubation. In addition to the desired oligosaccharide products derived from galactosyl acceptor, the composition furthermore contains undesired oligosaccharides derived from galactosylation of free leaving groups, e.g. allo-lactose and further galactosylated allo-lactose.

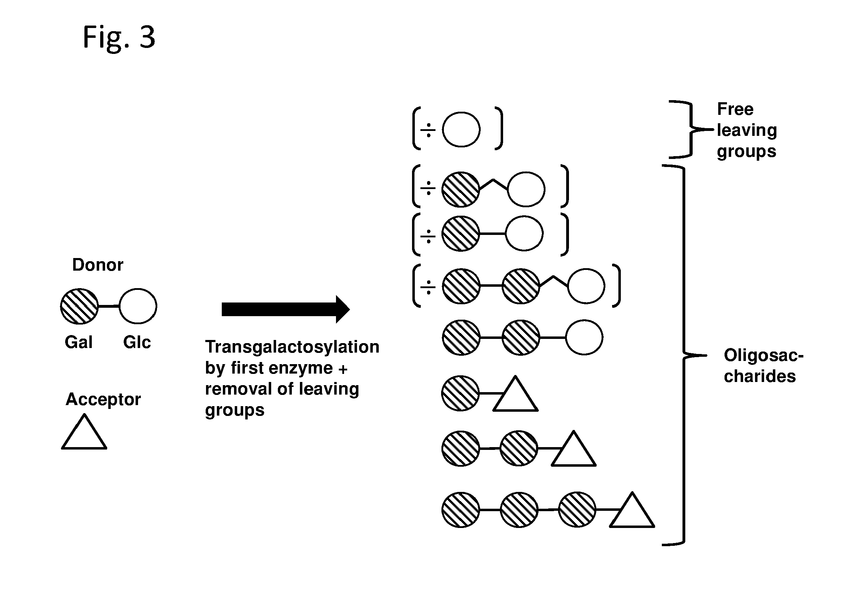

Figure 3 is a schematic illustration of some of the molecular species present in the galacto-oligosaccharide compositions when the free leaving groups are removed during incubation. Contrary to the galacto-oligosaccharide composition illustrated in Figure 2, the present galacto-oligosaccharide composition lacks (or has at least a significantly lower concentration of) free leaving groups, allo-lactose and oligosaccharides derived from allo-lactose.

Thus, an aspect of the invention relates to a method of producing a composition comprising one or more galacto-oligosaccharide(s), the method comprising the steps of:

a) providing a mixture comprising

- a galactosyl donor comprising a galactosyl group bound to a leaving group, which galactosyl donor has a molar weight of at most 350 g/mol,
- a galactosyl acceptor which is different from the galactosyl donor,
   said galactosyl acceptor is a saccharide or a sugar-alcohol, and
   wherein the molar ratio between the galactosyl acceptor and the galactosyl donor is at least 1:10, and wherein the mixture comprises at least 0.05 mol/L of the galactosyl acceptor,

b) providing a first enzyme, said first enzyme having beta-galactosidase activity, said first enzyme contacting the mixture, and
c) incubating the mixture and the first enzyme, thereby allowing the first enzyme to release the leaving group of the galactosyl donor and transfer the galactosyl group of the galactosyl donor to the galactosyl acceptor, thus forming the galacto-oligosaccharide, step c) furthermore comprising removing from the incubating mixture, i.e. during incubation, a leaving group released from the galactosyl donor, thereby obtaining the composition comprising the one or more galacto-oligosaccharide(s).

The term "removing a leaving group released from the galactosyl donor" should be understood as physical removal of free leaving groups from the incubating mixture and/or conversion of free leaving groups into one or more other chemical species which may still be present in the incubating mixture. It is preferred that such chemical species do not act as galactosyl acceptors, or at least that they are a less efficient galactosyl acceptor than the free leaving group.
This invention opens up for cheap and efficient production of complex galacto-oligosaccharide compositions in high yield. The present invention furthermore appears to reduce the galactosylation of free leaving groups released from of the galactosyl donor and well as the self-galactosylation of the galactosyl donor, which both result in undesired by-products which are expensive to remove from the composition.
Preferably, the first enzyme has transgalactosylating activity in addition to beta-galactosidase activity. It may also be preferred that the first enzyme has a T-value of at most 0.9.
In the context of the present invention the term "transgalactosylating activity" of a beta-galactosidase enzyme relates to the ability of the enzyme to transfer a galactosyl group from a donor molecule, e.g. a lactose molecule, to a non-water molecule, e.g. another lactose molecule.
The T-value is a measure of the transgalactosylating efficiency of a beta-galactosidase enzyme using lactose both as galactosyl donor and acceptor. The determination of the T-value of a beta-galactosidase enzyme is performed according to the assay and the formula described in Example 2. The T-value is calculated using the formula:

$$\text{T-value} = \frac{\text{amount of produced galactose (in mol)}}{\text{amount of used lactose (in mol)}}$$

A lactase enzyme without any transgalactosylating activity will produce one mol galactose for each used mol lactose and would have a T-value of 1. A beta-galactosidase having an extremely high transgalactosylating activity would use nearly all the galactosyl groups from the lactose for transgalactosylating instead of generating galactose, and would consequently have a T-value near 0.
Yet an aspect of the invention relates to a composition comprising one or more galacto-oligosaccharide(s), which composition is obtainable by the method as described herein.
Additional objects and advantages of the invention are described below.

**BRIEF DESCRIPTION OF THE FIGURES**

Figure 1 shows a schematic illustration of the types of reactions involved in transgalactosylation.

Figure 2 shows a schematic representation of the reaction products obtained when the free leaving groups are not used.

Figure 3 shows a schematic representation of the reaction products obtained when the free leaving groups are not used.

## DETAILED DESCRIPTION OF THE INVENTION

As mentioned, an aspect of the invention relates to a method of producing a composition comprising one or more galacto-oligosaccharide(s), the method comprising the steps of:

a) providing a mixture comprising

- a galactosyl donor comprising a galactosyl group bound to a leaving group, which galactosyl donor has a molar weight of at most 350 g/mol,
- a galactosyl acceptor which is different from the galactosyl donor,
  said galactosyl acceptor is a saccharide or a sugar-alcohol, and
  wherein the molar ratio between the galactosyl acceptor and the galactosyl donor is at least 1:10, and wherein the mixture comprises at least 0.05 mol/L of the galactosyl acceptor,

b) providing a first enzyme, said first enzyme having beta-galactosidase activity, said first enzymes contacting the mixture, and

c) incubating the mixture and the first enzyme, thereby allowing the first enzyme to release the leaving group of the galactosyl donor and transfer the galactosyl group of the galactosyl donor to the galactosyl acceptor, thus forming the galacto-oligosaccharide, step c) furthermore comprising removing from the incubating mixture, i.e. during incubation, a leaving group released from the galactosyl donor, thereby obtaining the composition comprising the one or more galacto-oligosaccharide(s).

In the context of the present invention, the term "glycosyl group" relates to a group obtained by removing one or two hydroxyl groups from a monosaccharide or a lower oligosaccharide, such as a di- or tri-saccharide, or from corresponding sugar-alcohols. The term is used herein to describe various building blocks of galactosyl donors, galactosyl acceptors and oligosaccharides.

The abbreviations of the most common saccharides and their corresponding glycosyl groups are shown below.

| Saccharide | Abbreviation | Name of glycosyl group |
|---|---|---|
| Glucose | Glc | glucosyl |
| Galactose | Gal | galactosyl |
| fucose | Fuc | fucosyl |
| mannose | Man | mannosyl |
| xylose | Xyl | xylosyl |
| N-acetylgalactosamine | GalNAc | N-acetylgalactosaminyl |
| Lactose | Lac | lactosyl |

In the context of the present invention, the term "oligosaccharide" relates to a molecule comprising at least two glycosyl groups, and preferably at least three, which may be different or the same type. The at least two glycosyl groups are preferably bound via an O-glycosylic bond. An oligosaccharide may be a linear chain of glycosyl groups or it may have a branched structure. Oligosaccharides may e.g. be represented as a stoichiometric formula, e.g. $(Gal)_3Glc$, or as general formulas, e.g. Gal-Gal-Gal-Glc, Gal-Gal-Glc-Gal, or Gal-(Gal-)Glc-Gal. The stoichiometric formulas provide information regarding which glycosyl groups an oligosaccharide, or a group of oligosaccharides, contains, but not the relative position of these, whereas the general formulas also contain general information regarding the relative positions of the glycosyl groups.

In the context of the present invention the term "homo-oligosaccharide" relates to an oligosaccharide containing only one type of glycosyl group. Examples of homo-oligosaccharides are Gal-Gal-Gal-Gal and Glc-Glc-Glc.

In the context of the present invention the term "hetero-oligosaccharide" relates to an oligosaccharide which contains different glycosyl groups, e.g. Gal-Gal-Glc, or Gal-Gal-Fuc.

In the context of the present invention, the prefix "galacto-" used together with the term "oligosaccharide" indicates that the oligosaccharide contains galactosyl groups as the repeating unit. The "homo-" or "hetero-" prefix may be used together with the "galacto-" prefix. Both Gal-Gal-Glc and Gal-Gal-Gal-Gal are galacto-oligosaccharides. Gal-Gal-Glc is a hetero-galacto-oligosaccharide and Gal-Gal-Gal-Gal is a homo-galacto-oligosaccharide.

In the context of the present invention, "X" represents a galactosyl acceptor as defined herein. "-X" represents the glycosyl group corresponding to the galactosyl acceptor, and particularly the glycosyl group bound to another group. "-" symbolises the bond. The glycosyl group is preferably bound via the 3-, 4-, 5- or 6-position of the glycosyl group, and preferably via an O-glycosylic bond.

In the context of the present invention, "Gal-" represents a galactosyl group bound to another group, preferably via the 1-position of the galactosyl group, and preferably via an O-glycosylic bond.

In the context of the present invention, "-Gal-" represents a galactosyl group bound to two other groups. The left bond is preferably made via the 4- or 6-position of the galactosyl group, and preferably via an O-glycosylic bond. The right bond is preferably made via the 1-position of the galactosyl group, and preferably via an O-glycosylic bond.

Bonds between two galactosyl groups are typically 1-4 or 1-6 bonds, and normally O-glycosylic bonds. A bond between a galactosyl group and a nitrogen-containing acceptor may alternatively be an N-glycosylic bond.

Method of the present invention is preferably a method of producing a composition comprising one or more galacto-oligosaccharide(s) using a galactosyl donor and a galactosyl acceptor, which one or more galacto-oligosaccharide(s) have the galactosyl acceptor as its reducing end.

In the context of the present invention the terms "method" and "process" are used interchangeably.

Step a) involves the provision of the mixture in which the oligosaccharides are to be produced.

The mixture is preferably a liquid mixture and may e.g. be an aqueous solution containing the galactosyl acceptor and the galactosyl donor.

In some embodiments of the invention the molar ratio between the galactosyl acceptor and the galactosyl donor of the mixture of step a) is at least 1:5, preferably at least 1:1, and even more preferably at least 5:1. For example, the molar ratio between the galactosyl acceptor and the galactosyl donor of the mixture of step a) may be at least 10: 1, such as at least 15: 1.

The molar ratio between the galactosyl acceptor and the galactosyl donor of the mixture of step a) may e.g. be in the range of 1:10-100:1.

In some embodiments of the invention the molar ratio between the galactosyl acceptor and the galactosyl donor of the mixture of step a) is in the range of 1:10-50:1, preferably in the range of 1:5-30:1, and even more preferably in the range of 1:1-20:1. For example, the molar ratio between the galactosyl acceptor and the galactosyl donor of the mixture of step a) may e.g. be in the range of 2:1-40:1, preferably in the range of 4:1-30:1, and even more preferably in the range of 10:1-25:1.

As mentioned, the galactosyl donors contain a galactosyl group covalently bound to a leaving group. The galactosyl group is preferably a β-D-galactopyranosyl group. Furthermore, the galactosyl group is preferably bound to the leaving group via an O-glycosidic bond from the 1-position of the galactosyl group.

The leaving group of the galactosyl donor may for example be a glycosyl group and/or a sugar-alcohol group. It is particularly preferred that the leaving group of the galactosyl donor is a glucosyl group, i.e. a glucose residue.

If the leaving group is a glycosyl group of a mono- or disaccharide or a corresponding sugar-alcohol, the galactosyl group is preferably bound to the leaving group via an O-glycosidic bond from the 1-position of the galactosyl group, which bond attaches to the 4-position of a monosaccharide-type leaving group or to the 4'-position of a disaccharide-type leaving group.

In the context of the present invention, the phrase "Y and/or X" means "Y" or "X" or "Y and X". Along the same line of logic, the phrase "$X_1$, $X_2$,..., $X_{1-1}$, and/or $X_i$" means "$X_1$" or "$X_2$" or "$X_{1-1}$" or "$X_i$" or any combination of the components : $X_1$, $X_2$,...$X_{i-1}$, and $X_i$.

In some embodiments of the invention the galactosyl donor has a molar weight of at most 1000 g/mol. For example, the galactosyl donor may have a molar weight of at most 500 g/mol. It may even be preferred that the galactosyl donor has a molar weight of at most 350 g/mol.

Disaccharides are a presently preferred type of galactosyl donor. Alternatively, or additionally, tri-saccharides may be used as galactosyl donors as well. Thus, it is envisioned that the mixture may contain a combination of different galactosyl donors.

In some preferred embodiments of the invention the galactosyl donor is lactose. Another example of a useful galactosyl donor is lactulose. Yet an example of a useful galactosyl donor is lactitol.

In the context of the present invention the term "lactose" relates to the disaccharide β-D-galactopyranosyl-(1→4)-D-glucose, which is also referred to as milk sugar, and which is the most predominant saccharide of bovine milk.

The galactosyl donor may be provided via any useful galactosyl donor source, both industrially refined sources, such as purified lactose, and/or natural sources, such as whey permeate, i.e. deproteinated whey prepared by ultrafiltration of whey.

The galactosyl acceptor may be any molecule capable of accepting a galactosyl group from the first enzyme and typically contains hydroxyl groups, and preferably alcoholic hydroxyl groups. The term "accepting" means that the galactosyl group of the donor should be covalently bound to the acceptor, e.g. via an O-glycosylic bond.

In some embodiments of the invention the galactosyl acceptor comprises one or more alcoholic hydroxyl group (s). For example, the galactosyl acceptor may be a polyol.

In the context of the present invention the term "polyol" relates to a molecule comprising at least two alcoholic hydroxyl groups.

In some preferred embodiments of the invention the galactosyl acceptor is not lactose. It may furthermore be preferred that the galactosyl acceptor is not glucose.

In some preferred embodiments of the invention the galactosyl acceptor is different from the galactosyl donor. It is particularly preferred to use a relatively cheap galactosyl donor, such as lactose, as galactosyl source, and a biologically interesting acceptor, such as fucose, as galactosyl acceptor.

In some embodiments of the invention the galactosyl acceptor is not lactose, galactose, or glucose.

In some embodiments of the invention the galactosyl acceptor is not glucose or oligosaccharides of the general formula $Gal-(Gal)_i-Glc$, where i is a non-negative integer, i.e. for example 0, 1, 2, 3, or 4.

In some embodiments of the invention the galactosyl acceptor is not galactose or oligosaccharides of the general formula $Gal-(Gal)_i-Gal$, where i is a non-negative integer.

Galactosyl acceptors having various molar weights may be used, but galactosyl acceptors having a molar weight of at least 100 g/mol are presently preferred.

In some embodiments of the invention the galactosyl acceptor has a molar weight of at most 1000 g/mol. For example, the galactosyl acceptor may have a molar weight of at most 500 g/mol. It may even be preferred that the galactosyl acceptor has a molar weight of at most 350 g/mol. The galactosyl acceptor may for example have a molar weight of at most 200 g/mol.

In some preferred embodiments of the invention the galactosyl acceptor is a saccharide. The galactosyl acceptor may for example be a mono-saccharide. Alternatively, the galactosyl acceptor may be a di-saccharide.

For example, the galactosyl acceptor may be a pentose. The galactosyl acceptor may e.g. be arabinose. Another example of a useful pentose is xylose. Yet an example of a useful pentose is ribose. The galactosyl acceptor may for example be a pentose selected from the group consisting of arabinose, xylose, and ribose.

Hexoses are another group of useful galactosyl acceptors. The galactosyl acceptor may e.g. be mannose. Another example of a useful hexose is galactose. Yet an example of a useful hexose is tagatose. A further example of a useful hexose is fructose. The galactosyl acceptor may for example be a hexose selected from the group consisting of mannose, galactose, tagatose, and fructose.

In some preferred embodiments of the invention the galactosyl acceptor is a deoxy-hexose. The galactosyl acceptor may for example be fucose, such as e.g. D-fucose, L-fucose, or a mixture thereof.

Alternatively, the galactosyl acceptor may be an oligosaccharide, such as e.g. a di-saccharide or a tri-saccharide. An example of a useful di-saccharide is maltose. Another example of a useful di-saccharide is lactulose.

Yet a useful group of galactosyl acceptors is saccharide derivatives.

In the context of the present invention the term "saccharide derivative" pertains to a saccharide containing one or more non-hydroxyl functional group(s). Examples of such functional groups are a carboxyl group, an amino group, an N-acetylamino group and/or a thiol group. Saccharides which contain an aldehyde group at the 1-position or a ketone group at the 2-position are not considered saccharide derivatives as such unless the saccharides comprise some of the non-hydroxyl functional groups mentioned above.

An example of a useful saccharide derivative is N-acetyl galactosamine. Another example of a useful saccharide derivative is sialic acid. Yet an example of a useful saccharide derivative is sialyl lactose. Thus, the galactosyl acceptor may be a saccharide derivative selected from the group consisting of N-acetyl galactosamine, sialic acid, and sialyl lactose.

Another group of useful galactosyl acceptors is sugar alcohols. Thus, in some embodiments of the invention the galactosyl acceptor is a sugar alcohol. Examples of useful sugar alcohols are sorbitol, xylitol, lactitol, and/or maltitol.

Contrary to the above-mentioned galactosyl acceptors, the present inventors have found that N-acetyl glucosamine and glucose are less efficient galactosyl acceptors. Thus, in some embodiments of the invention the galactosyl acceptor is not glucose or N-acetyl glucosamine.

The mixture may contain one or more further galactosyl acceptor(s) different from the first type of galactosyl acceptor. The different types of galactosyl acceptors of the mixture may e.g. be selected among the galactosyl acceptor types mentioned herein.

In some preferred embodiments of the invention the produced galactosylated acceptors act as a new type of galactosyl acceptor and can be galactosylated as well. In this way, galacto-oligosaccharides may be produced which have the stoichiometric formula $Gal_{i+1}X$, where i is a non-negative integer. Normally, the most predominant species are $GalX$, $Gal_2X$, and $Gal_3X$.

In other preferred embodiments of the invention the produced galactosylated acceptors act as a new type of galactosyl acceptor and can be galactosylated as well. In this way, galacto-oligosaccharides may be produced which have the general formula Gal-(Gal)$_i$-X, where i is a non-negative integer. Normally, the most predominant species are Gal-X, Gal-Gal-X, and Gal-Gal-Gal-X.

In some embodiments of the invention the mixture of step a) comprises the galactosyl donor in a concentration of at most 0.7 mol/L, preferably at most 0.4 mol/L, and even more preferably at most 0.2 mol/L. The mixture may e.g. comprise the galactosyl donor in a concentration in the range of 0.001-0.7 mol/L, preferably in the range of 0.01-0.5 mol/L, and even more preferred in the range of 0.02-0.2 mol/L.

Alternatively, the mixture of step a) may comprise the galactosyl donor in a concentration of at most 0.3 mol/L, preferably at most 0.1 mol/L, and even more preferably at most 0.05 mol/L. The mixture may e.g. comprise the galactosyl donor in a concentration in the range of 0.001-0.2 mol/L, preferably in the range of 0.005-0.1 mol/L, and even more preferred in the range of 0.01-0.05 mol/L.

It should be noted that galactosylated galactosyl acceptor and galactosylated galactosyl donor may to a limited extent act as a galactosyl donor, but galactosylated galactosyl acceptor and galactosylated galactosyl donor are not considered a galactosyl donor in the context of the present invention and do not contribute to the concentrations or ratios of galactosyl donor mentioned herein. The galactosyl acceptor may be used in a range of difference concentrations. It is, however, preferred to avoid saturating the mixture with the galactosyl acceptor since excess galactosyl acceptor normally has to be removed from the galacto-oligosaccharide-containing composition of the invention.

In some embodiments of the invention the mixture of step a) comprises the galactosyl acceptor in an amount of at least 0.05 mol/L, preferably at least 0.10 mol/L, and even more preferably at least 0.30 mol/L. Even higher concentrations of the galactosyl acceptor may be preferred, thus the mixture of step a) may e.g. comprise the galactosyl acceptor in an amount of at least 0.5 mol/L, preferably at least 0.7 mol/L, and even more preferably at least 1 mol/L.

The mixture may e.g. comprise the galactosyl acceptor in a concentration in the range of 0.05 mol/L - 5 mol/L, preferably in the range of 0.1 mol/L - 2 mol/L, and even more preferably in the range of 0.3 mol/L - 1 mol/L.

However, in some embodiments a relatively low concentration of the galactosyl acceptor is preferred in which case the mixture may e.g. comprise the galactosyl acceptor in a concentration of at most 2 mol/L, preferably at most 0.5 mol/L, and even more preferably at most 0.2 mol/L. For example, the mixture may comprise the galactosyl acceptor in a concentration in the range of 0.05 mol/L - 2 mol/L, preferably in the range of 0.06 mol/L - 1 mol/L, and even more preferably in the range of 0.08 mol/L - 0.8 mol/L.

In addition to galactosyl acceptor and galactosyl donor, the mixture may furthermore contain various additives for optimizing the conditions for the enzymatic reaction.

The mixture may for example contain one or more pH buffer(s) for adjusting the pH of the mixture to the pH optimum of the first enzyme. Alternatively, or in addition, the mixture may comprise water soluble salts containing one or more metal ions. Depending on the specific first enzyme, metal ions such as $Ca^{2+}$, $Zn^{2+}$, or $Mg^{2+}$ may e.g. be used. Note, however, that some first enzymes are insensitive to the presence of metal ions in the mixture.

Conventional methods of synthesising oligosaccharides often employ water-activity-lowering agents, such as e.g. glycerol, ethylene glycol, propylene glycol, polyethyleneglycol (PEG). The present invention advantageously makes it possible to perform efficient synthesis of galacto-oligosaccharides without the use of such water-activity-lowering agents. Thus, in some preferred embodiments of the invention the mixture contains water-activity-lowering agent in an amount of at most 5% by weight relative to the weight of the mixture, preferably at most 1% by weight, and even more preferably at most 0.1% by weight. For example, the mixture may contain water-activity-lowering agent in an amount of at most 0.05% by weight relative to the weight of the mixture.

The mixture of step a) or the ingredients forming the mixture may have been heat treated before the reaction with first enzyme to avoid microbial growth during the reaction. The usual heat treatment processes, such as pasteurisation (e.g. 72 degrees C for 15 seconds), high pasteurisation (e.g. 90 degrees C for 15 seconds), or UHT treatment (e.g. 140 degrees C for 4 seconds), may be used. Care should be taken when heat treating temperature labile enzymes.

Step b) involves the provision of a first enzyme, which preferably has beta-galactosidase activity, and preferably a T-value of at most 0.9. It should be noted that the method may furthermore involve the use of additional enzymes, e.g. enzymes having a different enzymatic activity than beta-galactosidase activity or transgalactosylating activity.

In the context of the present invention the term "beta-galactosidase activity" relates to enzymatic catalysis of the hydrolysis of terminal non-reducing β-D-galactose residues in β-D-galactosides, such as lactose. The first enzyme used in the invention preferably belongs to the class EC 3.2.1.23.

It is preferred that the first enzyme has a T-value of at most 0.9. In some embodiments of the invention, the T-value of the first enzyme is at most 0.8, preferably at most 0.7, and even more preferably at most 0.6. For example, the T-value of the first enzyme may be at most 0.5. Preferably the T-value of the first enzyme may be at most 0.4. It may even be more preferred that the T-value of the first enzyme is at most 0.3.

Even lower T-values may be preferred, such as at most 0.2.

Useful first enzymes may e.g. be derived from a peptide encoded by the DNA sequence of SEQ ID NO. 1. An example of such a peptide from which useful first enzymes may e.g. be derived is the peptide having the amino acid sequence of SEQ ID NO. 2.

SEQ ID NO. 1 and SEQ ID NO. 2 can be found in the PCT application WO 01/90,317 A2, where they are referred to as SEQ ID NO: 1 and SEQ ID NO: 2. Additionally, further useful first enzymes may be also be found in WO 01/90,317 A2.

In some preferred embodiments of the invention the first enzyme comprises an amino acid sequence having a sequence identity of at least 80% relative to the peptide of SEQ ID NO. 2. For example, the first enzyme may comprise an amino acid sequence having a sequence identity of at least 90% relative to the peptide of SEQ ID NO. 2, preferably at least 95%, and even more preferably at least 97.5%. In some instances the first enzyme may comprise an amino acid sequence having a sequence identity of at least 99% relative to the peptide of SEQ ID NO. 2.

In the context of the present invention the term "sequence identity" relates to a quantitative measure of the degree of identity between two amino acid sequences of equal length or between two nucleic acid sequences of equal length. If the two sequences to be compared are not of equal length, they must be aligned to the best possible fit. The sequence identity can be calculated as

$$(N_{ref}-N_{dif})*100)/(N_{ref}),$$

wherein $N_{dif}$ is the total number of non-identical residues in the two sequences when aligned, and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8). A gap is counted as non-identity of the specific residue(s), i.e. the DNA sequence AGTGTC will have a sequence identity of 75% with the DNA sequence AGTCAGTC (Ndif=2 and Nref=8). Sequence identity can for example be calculated using appropriate BLAST-programs, such as the BLASTp-algorithm provided by National Center for Biotechnology Information (NCBI), USA.

In other preferred embodiments of the invention the amino acid sequence of the first enzyme has a sequence identity of at least 80% relative to the peptide of SEQ ID NO. 2. For example, the amino acid sequence of the first enzyme may have a sequence identity of at least 90% relative to the peptide of SEQ ID NO. 2, preferably at least 95%, and even more preferably at least 97.5%. In some instances the amino acid sequence of the first enzyme may have a sequence identity of at least 99% relative to the peptide of SEQ ID NO. 2.

In some preferred embodiments of the invention the first enzyme comprises an amino acid sequence having a sequence identity of at least 80% relative to the amino acid sequence Val (33) to Gly (950) of SEQ ID NO. 2. For example, the first enzyme may comprise an amino acid sequence having a sequence identity of at least 90% relative to the amino acid sequence Val (33) to Gly (950) of SEQ ID NO. 2, preferably at least 95%, and even more preferably at least 97.5%. In some instances the first enzyme may comprise an amino acid sequence having a sequence identity of at least 99% relative to the amino acid sequence Val (33) to Gly (950) of SEQ ID NO. 2.

In other preferred embodiments of the invention the amino acid sequence of the first enzyme has a sequence identity of at least 80% relative to the amino acid sequence Val (33) to Gly (950) of SEQ ID NO. 2. For example, the amino acid sequence of the first enzyme may have a sequence identity of at least 90% relative to the amino acid sequence Val (33) to Gly (950) of SEQ ID NO. 2, preferably at least 95%, and even more preferably at least 97.5%. In some instances the amino acid sequence of the first enzyme may have a sequence identity of at least 99% relative to the amino acid sequence Val (33) to Gly (950) of SEQ ID NO. 2. Thus, the first enzyme may e.g. have the amino acid sequence Val (33) to Gly (950) of SEQ ID NO. 2.

In some preferred embodiments of the invention the first enzyme comprises an amino acid sequence having a sequence identity of at least 80% relative to the amino acid sequence Met (1) to Ile (1174) of SEQ ID NO. 2. For example, the first enzyme may comprise an amino acid sequence having a sequence identity of at least 90% relative to the amino acid sequence Met (1) to Ile (1174) of SEQ ID NO. 2, preferably at least 95%, and even more preferably at least 97.5%. In some instances the first enzyme may comprise an amino acid sequence having a sequence identity of at least 99% relative to the amino acid sequence Met (1) to Ile (1174) of SEQ ID NO. 2.

In other preferred embodiments of the invention the amino acid sequence of the first enzyme has a sequence identity of at least 80% relative to the amino acid sequence Met (1) to Ile (1174) of SEQ ID NO. 2. For example, the amino acid sequence of the first enzyme may have a sequence identity of at least 90% relative to the amino acid sequence Met (1) to Ile (1174) of SEQ ID NO. 2, preferably at least 95%, and even more preferably at least 97.5%. In some instances the amino acid sequence of the first enzyme may have a sequence identity of at least 99% relative to the amino acid sequence Met (1) to Ile (1174) of SEQ ID NO. 2.

In some presently preferred embodiments of the invention the first enzyme has the amino acid sequence Met (1) to Ile (1174) of SEQ ID NO. 2.

In some embodiments of the invention the first enzyme comprises an amino acid sequence having a sequence identity of at least 80% relative to the amino acid sequence Val (33) to Ile (1174) of SEQ ID NO. 2. For example, the first enzyme may comprise an amino acid sequence having a sequence identity of at least 90% relative to the amino acid sequence Val (33) to Ile (1174) of SEQ ID NO. 2, preferably at least 95%, and even more preferably at least 97.5%. In some instances the first enzyme may comprise an amino acid sequence having a sequence identity of at least 99% relative to the amino acid sequence Val (33) to Ile (1174) of SEQ ID NO. 2.

In other embodiments of the invention the amino acid sequence of the first enzyme may have a sequence identity of at least 80% relative to the amino acid sequence Val (33) to Ile (1174) of SEQ ID NO. 2. For example, the amino acid sequence of the first enzyme may have a sequence identity of at least 90% relative to the amino acid sequence Val (33) to Ile (1174) of SEQ ID NO. 2, preferably at least 95%, and even more preferably at least 97.5%. In some instances the amino acid sequence of the first enzyme may have a sequence identity of at least 99% relative to the amino acid sequence Val (33) to Ile (1174) of SEQ ID NO. 2. Thus, the first enzyme may e.g. have the amino acid sequence Val (33) to Ile (1174) of SEQ ID NO. 2.

In some presently preferred embodiments of the invention the first enzyme has the amino acid sequence Val (33) to Ile (1174) of SEQ ID NO. 2.

In some embodiments of the invention, the first enzyme may e.g. comprise an amino acid sequence having a sequence identity of at least 99% relative to an amino acid sequence shown in Table 1. The first enzyme may for example comprise an amino acid sequence shown in Table 1. Alternatively, the amino acid sequence of the first enzyme may have a sequence identity of at least 99% relative to an amino acid sequence shown in Table 1. The first enzyme may for example have an amino acid sequence shown in Table 1.

Table 1 Useful amino acid sequences (AAS).

| AAS No. | Position in SEQ ID NO. 2 | | AAS No. | Position in SEQ ID NO. 2 | | AAS No. | Position in SEQ ID NO. 2 | |
|---|---|---|---|---|---|---|---|---|
| | From | To | | From | To | | From | To |
| 1 | 25 | 1122 | 26 | 27 | 1122 | 51 | 30 | 1122 |
| 2 | 25 | 1132 | 27 | 27 | 1132 | 52 | 30 | 1132 |
| 3 | 25 | 1142 | 28 | 27 | 1142 | 53 | 30 | 1142 |
| 4 | 25 | 1152 | 29 | 27 | 1152 | 54 | 30 | 1152 |
| 5 | 25 | 1162 | 30 | 27 | 1162 | 55 | 30 | 1162 |
| 6 | 25 | 1167 | 31 | 27 | 1167 | 56 | 30 | 1167 |
| 7 | 25 | 1168 | 32 | 27 | 1168 | 57 | 30 | 1168 |
| 8 | 25 | 1169 | 33 | 27 | 1169 | 58 | 30 | 1169 |
| 9 | 25 | 1170 | 34 | 27 | 1170 | 59 | 30 | 1170 |
| 10 | 25 | 1171 | 35 | 27 | 1171 | 60 | 30 | 1171 |
| 11 | 25 | 1172 | 36 | 27 | 1172 | 61 | 30 | 1172 |
| 12 | 25 | 1173 | 37 | 27 | 1173 | 62 | 30 | 1173 |
| 13 | 25 | 1174 | 38 | 27 | 1174 | 63 | 30 | 1174 |
| 14 | 25 | 1175 | 39 | 27 | 1175 | 64 | 30 | 1175 |
| 15 | 25 | 1176 | 40 | 27 | 1176 | 65 | 30 | 1176 |
| 16 | 25 | 1177 | 41 | 27 | 1177 | 66 | 30 | 1177 |
| 17 | 25 | 1178 | 42 | 27 | 1178 | 67 | 30 | 1178 |
| 18 | 25 | 1179 | 43 | 27 | 1179 | 68 | 30 | 1179 |
| 19 | 25 | 1180 | 44 | 27 | 1180 | 69 | 30 | 1180 |
| 20 | 25 | 1181 | 45 | 27 | 1181 | 70 | 30 | 1181 |
| 21 | 25 | 1186 | 46 | 27 | 1186 | 71 | 30 | 1186 |
| 22 | 25 | 1196 | 47 | 27 | 1196 | 72 | 30 | 1196 |
| 23 | 25 | 1206 | 48 | 27 | 1206 | 73 | 30 | 1206 |
| 24 | 25 | 1216 | 49 | 27 | 1216 | 74 | 30 | 1216 |
| 25 | 25 | 1226 | 50 | 27 | 1226 | 75 | 30 | 1226 |

In some embodiments of the invention, the enzyme may e.g. comprise an amino acid sequence having a sequence identity of at least 99% relative to an amino acid sequence shown in Table 2. The enzyme may for example comprise an amino acid sequence shown in Table 2. Alternatively, the amino acid sequence of the enzyme may have a sequence identity of at least 99% relative to an amino acid sequence shown in Table 2. The enzyme may for example have an amino acid sequence shown in Table 2.

Table 2 Useful amino acid sequences (AAS).

| AAS No. | Position in SEQ ID NO. 2 | | AAS No. | Position in SEQ ID NO. 2 | | AAS No. | Position in SEQ ID NO. 2 | |
|---|---|---|---|---|---|---|---|---|
| | From | To | | From | To | | From | To |
| 76 | 31 | 1122 | 101 | 32 | 1122 | 126 | 33 | 1122 |
| 77 | 31 | 1132 | 102 | 32 | 1132 | 127 | 33 | 1132 |
| 78 | 31 | 1142 | 103 | 32 | 1142 | 128 | 33 | 1142 |
| 79 | 31 | 1152 | 104 | 32 | 1152 | 129 | 33 | 1152 |
| 80 | 31 | 1162 | 105 | 32 | 1162 | 130 | 33 | 1162 |
| 81 | 31 | 1167 | 106 | 32 | 1167 | 131 | 33 | 1167 |
| 82 | 31 | 1168 | 107 | 32 | 1168 | 132 | 33 | 1168 |
| 83 | 31 | 1169 | 108 | 32 | 1169 | 133 | 33 | 1169 |
| 84 | 31 | 1170 | 109 | 32 | 1170 | 134 | 33 | 1170 |
| 85 | 31 | 1171 | 110 | 32 | 1171 | 135 | 33 | 1171 |
| 86 | 31 | 1172 | 111 | 32 | 1172 | 136 | 33 | 1172 |
| 87 | 31 | 1173 | 112 | 32 | 1173 | 137 | 33 | 1173 |
| 88 | 31 | 1174 | 113 | 32 | 1174 | 138 | 33 | 1174 |
| 89 | 31 | 1175 | 114 | 32 | 1175 | 139 | 33 | 1175 |
| 90 | 31 | 1176 | 115 | 32 | 1176 | 140 | 33 | 1176 |
| 91 | 31 | 1177 | 116 | 32 | 1177 | 141 | 33 | 1177 |
| 92 | 31 | 1178 | 117 | 32 | 1178 | 142 | 33 | 1178 |
| 93 | 31 | 1179 | 118 | 32 | 1179 | 143 | 33 | 1179 |
| 94 | 31 | 1180 | 119 | 32 | 1180 | 144 | 33 | 1180 |
| 95 | 31 | 1181 | 120 | 32 | 1181 | 145 | 33 | 1181 |
| 96 | 31 | 1186 | 121 | 32 | 1186 | 146 | 33 | 1186 |
| 97 | 31 | 1196 | 122 | 32 | 1196 | 147 | 33 | 1196 |
| 98 | 31 | 1206 | 123 | 32 | 1206 | 148 | 33 | 1206 |
| 99 | 31 | 1216 | 124 | 32 | 1216 | 149 | 33 | 1216 |
| 100 | 31 | 1226 | 125 | 32 | 1226 | 150 | 33 | 1226 |

In some embodiments of the invention, the enzyme may e.g. comprise an amino acid sequence having a sequence identity of at least 99% relative to an amino acid sequence shown in Table 3. The enzyme may for example comprise an amino acid sequence shown in Table 3. Alternatively, the amino acid sequence of the enzyme may have a sequence identity of at least 99% relative to an amino acid sequence shown in Table 3. The enzyme may for example have an amino acid sequence shown in Table 3.

Table 3 Useful amino acid sequences (AAS).

| AAS No. | Position in SEQ ID NO. 2 | | AAS No. | Position in SEQ ID NO. 2 | | AAS No. | Position in SEQ ID NO. 2 | |
|---|---|---|---|---|---|---|---|---|
| | From | To | | From | To | | From | To |
| 151 | 34 | 1122 | 176 | 35 | 1122 | 201 | 36 | 1122 |
| 152 | 34 | 1132 | 177 | 35 | 1132 | 202 | 36 | 1132 |
| 153 | 34 | 1142 | 178 | 35 | 1142 | 203 | 36 | 1142 |
| 154 | 34 | 1152 | 179 | 35 | 1152 | 204 | 36 | 1152 |
| 155 | 34 | 1162 | 180 | 35 | 1162 | 205 | 36 | 1162 |
| 156 | 34 | 1167 | 181 | 35 | 1167 | 206 | 36 | 1167 |
| 157 | 34 | 1168 | 182 | 35 | 1168 | 207 | 36 | 1168 |
| 158 | 34 | 1169 | 183 | 35 | 1169 | 208 | 36 | 1169 |
| 159 | 34 | 1170 | 184 | 35 | 1170 | 209 | 36 | 1170 |
| 160 | 34 | 1171 | 185 | 35 | 1171 | 210 | 36 | 1171 |
| 161 | 34 | 1172 | 186 | 35 | 1172 | 211 | 36 | 1172 |

(continued)

| AAS No. | Position in SEQ ID NO. 2 | | AAS No. | Position in SEQ ID NO. 2 | | AAS No. | Position in SEQ ID NO. 2 | |
|---|---|---|---|---|---|---|---|---|
| | From | To | | From | To | | From | To |
| 162 | 34 | 1173 | 187 | 35 | 1173 | 212 | 36 | 1173 |
| 163 | 34 | 1174 | 188 | 35 | 1174 | 213 | 36 | 1174 |
| 164 | 34 | 1175 | 189 | 35 | 1175 | 214 | 36 | 1175 |
| 165 | 34 | 1176 | 190 | 35 | 1176 | 215 | 36 | 1176 |
| 166 | 34 | 1177 | 191 | 35 | 1177 | 216 | 36 | 1177 |
| 167 | 34 | 1178 | 192 | 35 | 1178 | 217 | 36 | 1178 |
| 168 | 34 | 1179 | 193 | 35 | 1179 | 218 | 36 | 1179 |
| 169 | 34 | 1180 | 194 | 35 | 1180 | 219 | 36 | 1180 |
| 170 | 34 | 1181 | 195 | 35 | 1181 | 220 | 36 | 1181 |
| 171 | 34 | 1186 | 196 | 35 | 1186 | 221 | 36 | 1186 |
| 172 | 34 | 1196 | 197 | 35 | 1196 | 222 | 36 | 1196 |
| 173 | 34 | 1206 | 198 | 35 | 1206 | 223 | 36 | 1206 |
| 174 | 34 | 1216 | 199 | 35 | 1216 | 224 | 36 | 1216 |
| 175 | 34 | 1226 | 200 | 35 | 1226 | 225 | 36 | 1226 |

In some embodiments of the invention, the enzyme may e.g. comprise an amino acid sequence having a sequence identity of at least 99% relative to an amino acid sequence shown in Table 4. The enzyme may for example comprise an amino acid sequence shown in Table 4. Alternatively, the amino acid sequence of the enzyme may have a sequence identity of at least 99% relative to an amino acid sequence shown in Table 4. The enzyme may for example have an amino acid sequence shown in Table 4.

Table 4 Useful amino acid sequences (AAS).

| AAS No. | Position in SEQ ID NO. 2 | | AAS No. | Position in SEQ ID NO. 2 | |
|---|---|---|---|---|---|
| | From | To | | From | To |
| 226 | 39 | 1122 | 251 | 42 | 1122 |
| 227 | 39 | 1132 | 252 | 42 | 1132 |
| 228 | 39 | 1142 | 253 | 42 | 1142 |
| 229 | 39 | 1152 | 254 | 42 | 1152 |
| 230 | 39 | 1162 | 255 | 42 | 1162 |
| 231 | 39 | 1167 | 256 | 42 | 1167 |
| 232 | 39 | 1168 | 257 | 42 | 1168 |
| 233 | 39 | 1169 | 258 | 42 | 1169 |
| 234 | 39 | 1170 | 259 | 42 | 1170 |
| 235 | 39 | 1171 | 260 | 42 | 1171 |
| 236 | 39 | 1172 | 261 | 42 | 1172 |
| 237 | 39 | 1173 | 262 | 42 | 1173 |
| 238 | 39 | 1174 | 263 | 42 | 1174 |
| 239 | 39 | 1175 | 264 | 42 | 1175 |
| 240 | 39 | 1176 | 265 | 42 | 1176 |
| 241 | 39 | 1177 | 266 | 42 | 1177 |
| 242 | 39 | 1178 | 267 | 42 | 1178 |
| 243 | 39 | 1179 | 268 | 42 | 1179 |
| 244 | 39 | 1180 | 269 | 42 | 1180 |
| 245 | 39 | 1181 | 270 | 42 | 1181 |
| 246 | 39 | 1186 | 271 | 42 | 1186 |
| 247 | 39 | 1196 | 272 | 42 | 1196 |
| 248 | 39 | 1206 | 273 | 42 | 1206 |
| 249 | 39 | 1216 | 274 | 42 | 1216 |

(continued)

| AAS No. | Position in SEQ ID NO. 2 | | AAS No. | Position in SEQ ID NO. 2 | |
|---|---|---|---|---|---|
| | From | To | | From | To |
| 250 | 39 | 1226 | 275 | 42 | 1226 |

In some embodiments of the invention, the first enzyme may e.g. comprise an amino acid sequence having a sequence identity of at least 99% relative to an amino acid sequence shown in Table 5. The first enzyme may for example comprise an amino acid sequence shown in Table 5. Alternatively, the amino acid sequence of the first enzyme may have a sequence identity of at least 99% relative to an amino acid sequence shown in Table 5. The first enzyme may for example have an amino acid sequence shown in Table 5.

Table 5 Useful amino acid sequences (AAS).

| Enzyme no. | Position in SEQ ID NO. 2 | | Enzyme no. | Position in SEQ ID NO. 2 | | Enzyme no. | Position in SEQ ID NO. 2 | |
|---|---|---|---|---|---|---|---|---|
| | From | To | | From | To | | From | To |
| 276 | 31 | 898 | 301 | 33 | 898 | 326 | 37 | 898 |
| 277 | 31 | 908 | 302 | 33 | 908 | 327 | 37 | 908 |
| 278 | 31 | 918 | 303 | 33 | 918 | 328 | 37 | 918 |
| 279 | 31 | 928 | 304 | 33 | 928 | 329 | 37 | 928 |
| 280 | 31 | 938 | 305 | 33 | 938 | 330 | 37 | 938 |
| 281 | 31 | 943 | 306 | 33 | 943 | 331 | 37 | 943 |
| 282 | 31 | 944 | 307 | 33 | 944 | 332 | 37 | 944 |
| 283 | 31 | 945 | 308 | 33 | 945 | 333 | 37 | 945 |
| 284 | 31 | 946 | 309 | 33 | 946 | 334 | 37 | 946 |
| 285 | 31 | 947 | 310 | 33 | 947 | 335 | 37 | 947 |
| 286 | 31 | 948 | 311 | 33 | 948 | 336 | 37 | 948 |
| 287 | 31 | 949 | 312 | 33 | 949 | 337 | 37 | 949 |
| 288 | 31 | 950 | 313 | 33 | 950 | 338 | 37 | 950 |
| 289 | 31 | 951 | 314 | 33 | 951 | 339 | 37 | 951 |
| 290 | 31 | 952 | 315 | 33 | 952 | 340 | 37 | 952 |
| 291 | 31 | 953 | 316 | 33 | 953 | 341 | 37 | 953 |
| 292 | 31 | 954 | 317 | 33 | 954 | 342 | 37 | 954 |
| 293 | 31 | 955 | 318 | 33 | 955 | 343 | 37 | 955 |
| 294 | 31 | 956 | 319 | 33 | 956 | 344 | 37 | 956 |
| 295 | 31 | 957 | 320 | 33 | 957 | 345 | 37 | 957 |
| 296 | 31 | 962 | 321 | 33 | 962 | 346 | 37 | 962 |
| 297 | 31 | 972 | 322 | 33 | 972 | 347 | 37 | 972 |
| 298 | 31 | 982 | 323 | 33 | 982 | 348 | 37 | 982 |
| 299 | 31 | 992 | 324 | 33 | 992 | 349 | 37 | 992 |
| 300 | 31 | 1002 | 325 | 33 | 1002 | 350 | 37 | 1002 |

Other examples of useful first enzymes having transgalactosylating activity can be found in WO 2011/120993 A1, which is incorporated herein by reference.

In some embodiments of the invention the first enzyme may contain one or more glycosylated amino acid(s). Alternatively, or in addition, the first enzyme may contain one or more phosphorylated amino acid(s). Alternatively, none of the amino acids of the first enzyme are glycosylated or phosphorylated.

In some preferred embodiments of the invention the first enzyme comprises at least two sub-units, each sub-unit consisting of a first enzyme as defined above.

The first enzyme preferably contacts the mixture and is thereby brought into contact with both the galactosyl acceptor and the galactosyl donor.

In some embodiments of the invention the mixture comprises the first enzyme and/or the second enzyme. The first enzyme and/or the second enzyme may e.g. be present in the mixture in dissolved form, e.g. as single enzyme molecules or as soluble aggregate of enzyme molecules.

In other embodiments of the invention the first enzyme and/or the second enzyme is/are separate from the mixture, but brought in contact with the galactosyl acceptor and the galactosyl donor by contacting the first enzyme and/or the second enzyme with the mixture. For example, first enzyme and/or second enzyme immobilised on a stationary solid phase may be used. Examples of useful stationary solid phases are e.g. a filter, a packed bed of first enzyme-containing particles, or similar structures.

Alternatively, the solid phase may e.g. be a free flowing, particulate solid phase, e.g. organic or inorganic beads, forming part of the mixture.

Details relating to the industrial use of enzymes including immobilisation techniques and suitable solid phase types can be found in Buchholz (2005), which is incorporated herein by reference for all purposes.

The first enzyme is preferably used in a sufficient activity to obtain an acceptable yield of galacto-oligosaccharides. The optimal activity depends on the specific implementation of the process and can easily be determined by the person skilled in the art.

If a high turn-over of galactosyl donor and a high yield of galacto-oligosaccharide is required, it may be preferred to use the first enzyme in a relatively high activity. For example, the activity of the first enzyme may be such that the turn-over of the galactosyl donor is at least 0.02 mol/(L*h), preferably at least 0.2 mol/(L*h), and even more preferably at least 2 mol/(L*h).

The enzymatic reaction takes place during the incubation of step c). As soon as the mixture is exposed to the right conditions, which may be almost immediately when the galactosyl acceptor and the galactosyl donor are brought into contact with the first enzyme, the transgalactosylation usually starts, and in some embodiments of the invention steps b) and c) occur simultaneously.

The first enzyme is capable of releasing the leaving group of the galactosyl donor and transferring the galactosyl group of the galactosyl donor to the galactosyl acceptor. For example, if the galactosyl donor is lactose, glucose is released and the galactosyl group is transferred to the acceptor. The first enzyme acts as catalyst during the enzymatic reaction.

In some preferred embodiments of the invention the first enzyme furthermore transfers galactosyl groups to already galactosylated galactosyl acceptors, thereby generating galactosyl acceptors containing two, three or even more galactosyl groups.

The pH of the incubating mixture is preferably near the optimum pH of the first enzyme. In some embodiments of the invention the pH of the incubating mixture during step c) is in the range of pH 3-9. For example, the pH of the incubating mixture during step c) may be in the range of pH 4-8, such as in the range of pH 5-7.5.

Similar to the pH, the temperature of the incubating mixture is preferably adjusted to the optimum temperature of the used first enzyme. In some embodiments of the invention the temperature of the incubating mixture of step c) is in the range of 10-80 degrees C. The temperature of the incubating mixture may e.g. be in the range of 20-70 degrees C, preferably in the range of 25-60 degrees C, and even more preferably in the range of 30-50 degrees C.

In the context of the present invention, the term "optimum pH of the first enzyme" relates to the pH where the first enzyme has the highest transgalactosylating activity. Along the same lines, the term "optimum temperature of the first enzyme" relates to the temperature where the first enzyme has the highest transgalactosylating activity.

Step c) may furthermore involve stirring the incubating mixture.

The removal of free leaving groups takes place during the incubation of step c). The removal may for example start immediately when step c) starts or it may be postponed until a significant amount of free leaving groups start building up in the incubating mixture.

The free leaving groups may for example be removed from the incubating mixture by microorganisms present in the incubating mixture.

Thus, in some embodiments of the invention, the method furthermore comprises providing a microorganism which is capable of converting free leaving groups released from the galactosyl donor, and allowing said microorganism, during incubation, to remove a leaving group released from the galactosyl donor.

Useful microorganisms are preferably able to selectively remove the free leaving groups from the incubating mixture and e.g. metabolise or convert these into conversion products that interfere less with the synthesis of galacto-oligosaccharides than the leaving group.

In some preferred embodiments of the invention, the removal rate of the microorganism relative to the free leaving group is at least 10 times higher than its removal rate relative to the galactosyl acceptor.

For example, the removal rate of the microorganism relative to the free leaving group may be at least $10^2$ times higher than its removal rate relative to the galactosyl acceptor, preferably at least $10^3$ times higher, and even more preferred at least $10^4$ times higher than its removal rate relative to the galactosyl acceptor. The removal rate of the microorganism relative to the free leaving group may e.g. be at least $10^5$ times higher than its removal rate relative to the galactosyl acceptor. It may even be preferred that the removal rate of the microorganism relative to the free leaving group is at least $10^6$ times higher, and even more preferred at least $10^7$ times higher than its removal rate relative to the galactosyl acceptor.

In the context of the present invention, the term "removal rate" pertains to the number of moles of free leaving group, donor, acceptor or mono-galactosylated acceptor that the microorganism is capable of removing from the incubating mixture per minute.

In some preferred embodiments of the invention, the removal rate of the microorganism relative to the free leaving group is at least 10 times higher than its removal rate relative to the galactosyl donor.

For example, the removal rate of the microorganism relative to the free leaving group may be at least $10^2$ times higher than its removal rate relative to the galactosyl donor, preferably at least $10^3$ times higher, and even more preferred at least $10^4$ times higher than its removal rate relative to the galactosyl donor. The removal rate of the microorganism relative to the free leaving group may e.g. be at least $10^5$ times higher than its removal rate relative to the galactosyl donor. It may even be preferred that the removal rate of the microorganism relative to the free leaving group is at least $10^6$ times higher, and even more preferred at least $10^7$ times higher than its removal rate relative to the galactosyl donor.

In some preferred embodiments of the invention, the removal rate of the microorganism relative to the free leaving group is at least 10 times higher than its removal rate relative to the mono-galactosylated galactosyl acceptor.

For example, the removal rate of the microorganism relative to the free leaving group may be at least $10^2$ times higher than its removal rate relative to the mono-galactosylated galactosyl acceptor, preferably at least $10^3$ times higher, and even more preferred at least $10^4$ times higher than its removal rate relative to the mono-galactosylated galactosyl acceptor. The removal rate of the microorganism relative to the free leaving group may e.g. be at least $10^5$ times higher than its removal rate relative to the mono-galactosylated galactosyl acceptor. It may even be preferred that the removal rate of the microorganism relative to the free leaving group is at least $10^6$ times higher, and even more preferred at least $10^7$ times higher than its removal rate relative to the mono-galactosylated galactosyl acceptor.

The microorganism is preferably a yeast or a bacterium.

Saccharomyces cerevisiae is an example of a useful yeast, and useful bacteria could for example be Lac Z-negative E.coli strains or other bacteria which can metabolise glucose but not lactose.

Alternatively, or additionally, free leaving groups may be removed from the incubating mixture by means of specific enzymes that convert the leaving groups into other chemical species.

Thus, in some preferred embodiments of the invention, the method furthermore comprises providing a second enzyme which is capable of converting free leaving groups released from the galactosyl donor and allowing said second enzyme, during incubation, to convert a leaving group released from the galactosyl donor.

The term "converting free leaving groups" pertains to the process of converting the free leaving groups into chemical species which preferably are poorer galactosyl acceptors than the free leaving groups as such. The conversion may involve degradation of the free leaving group into several smaller chemical species or it may simply involve a transformation of the free leaving group into a different chemical species.

The present invention may for example pertain to a method of producing a composition comprising one or more galacto-oligosaccharide(s), the method comprising the steps of:

a) providing a mixture comprising

- a galactosyl donor comprising a galactosyl group bound to a leaving group, which galactosyl donor has a molar weight of at most 350 g/mol,
- a galactosyl acceptor which is different from the galactosyl donor,

said galactosyl acceptor is a saccharide or a sugar-alcohol, and
wherein the molar ratio between the galactosyl acceptor and the galactosyl donor is at least 1:10, and wherein the mixture comprises at least 0.05 mol/L of the galactosyl acceptor,

b) providing a first enzyme and a second enzyme, said first enzyme having beta-galactosidase activity and a T-value of at most 0.9, said second enzyme being capable of converting free leaving groups released from the galactosyl donor, and said first and second enzymes contacting the mixture, and

c) allowing the first enzyme to release the leaving group of the galactosyl donor and transferring the galactosyl group of the galactosyl donor to the galactosyl acceptor, thus forming the galacto-oligosaccharide, and allowing the second enzyme to convert a leaving group released from the galactosyl donor, thereby obtaining the composition comprising the galacto-oligosaccharide.

The second enzyme may for example have hexose oxidase activity, i.e. belonging to the enzyme class EC 1.1.3.5 and having the ability to catalyze the oxidation of beta-D-glucose with $O_2$ to form D-glucono-1,5-lactone and hydrogen peroxide.

It is even more preferred that the second enzyme has glucose oxidase activity, i.e. belonging to the enzyme class EC 1.1.3.4 and having the ability to catalyze the oxidation of beta-D-glucose with $O_2$ to form D-glucono-1,5-lactone and hydrogen peroxide without significant oxidation of glucose-containing disaccharides such as lactose.

In an aqueous, acidic environment, D-glucono-1,5-lactone typically hydrolyses to form gluconic acid.

Alternatively, the second enzymes may have hexokinase activity (EC 2.7.1.1) or glucokinase activity (EC 2.7.1.2).

Other useful second enzymes may e.g. be found in handbooks which are available to the person skilled in the art.

It is preferred that the second enzyme is selective in its conversion of the free leaving groups and only to a limited extend, and preferably not at all, converts the galactosyl donor, the galactosyl acceptor and/or the galacto-oligosaccharides. Thus, in some preferred embodiments of the invention, the specificity constant of the second enzyme relative to the free leaving group is at least 10 times higher than its specificity constant relative to the galactosyl acceptor.

For example, the specificity constant of the second enzyme relative to the free leaving group may be at least $10^2$ times higher than its specificity constant relative to the galactosyl acceptor, preferably at least $10^3$ times higher, and even more preferred at least $10^4$ times higher than its specificity constant relative to the galactosyl acceptor. The specificity constant of the second enzyme relative to the free leaving group may e.g. be at least $10^5$ times higher than its specificity constant relative to the galactosyl acceptor. It may even be preferred that the specificity constant of the second enzyme relative to the free leaving group is at least $10^6$ times higher, and even more preferred at least $10^7$ times higher than its specificity constant relative to the galactosyl acceptor.

The term "specificity constant" is determined as $k_{cat}/K_m$ and incorporates the rate constants for all steps in the reaction. Because the specificity constant reflects both affinity and catalytic ability, it is useful for comparing different enzymes against each other, or the same enzyme with different substrates. The theoretical maximum for the specificity constant is called the diffusion limit and is about $10^8$ to $10^9$ ($M^{-1}$ $s^{-1}$). At this point every collision of the enzyme with its substrate will result in catalysis, and the rate of product formation is not limited by the reaction rate but by the diffusion rate.

$k_{cat}$ and $K_m$ of the second enzyme is determined at 37 degrees using 50 mM $Na_3PO_4$ buffer adjusted to pH 6.5. The buffer furthermore contains the salts and co-factors which are required for optimal performance of the enzyme. In some preferred embodiments of the invention, the specificity constant of the second enzyme relative to the free leaving group is at least 10 times higher than its specificity constant relative to the galactosyl donor.

For example, the specificity constant of the second enzyme relative to the free leaving group may be at least $10^2$ times higher than its specificity constant relative to the galactosyl donor, preferably at least $10^3$ times higher, and even more preferred at least $10^4$ times higher than its specificity constant relative to the galactosyl donor. The specificity constant of the second enzyme relative to the free leaving group may e.g. be at least $10^5$ times higher than its specificity constant relative to the galactosyl donor. It may even be preferred that the specificity constant of the second enzyme relative to the free leaving group is at least $10^6$ times higher, and even more preferred at least $10^7$ times higher than its specificity constant relative to the galactosyl donor.

In some preferred embodiments of the invention, the specificity constant of the second enzyme relative to the free leaving group is at least 10 times higher than its specificity constant relative to the mono-galactosylated galactosyl acceptor (Gal-X).

For example, the specificity constant of the second enzyme relative to the free leaving group may be at least $10^2$ times higher than its specificity constant relative to the mono-galactosylated galactosyl acceptor, preferably at least $10^3$ times higher, and even more preferred at least $10^4$ times higher than its specificity constant relative to the mono-galactosylated galactosyl acceptor. The specificity constant of the second enzyme relative to the free leaving group may e.g. be at least $10^5$ times higher than its specificity constant relative to the mono-galactosylated galactosyl acceptor. It may even be preferred that the specificity constant of the second enzyme relative to the free leaving group is at least $10^6$ times higher, and even more preferred at least $10^7$ times higher than its specificity constant relative to the mono-galactosylated galactosyl acceptor.

In some preferred embodiments of the invention, the specificity constant of the second enzyme relative to the free leaving group is:

- at least 10 times higher than its specificity constant relative to the galactosyl donor,
- at least 10 times higher than its specificity constant relative to the galactosyl acceptor, and
- at least 10 times higher than its specificity constant relative to the mono-galactosylated galactosyl acceptor.

For example, the specificity constant of the second enzyme relative to the free leaving group may be:

- at least $10^2$ times higher than its specificity constant relative to the galactosyl donor,
- at least $10^2$ times higher than its specificity constant relative to the galactosyl acceptor, and
- at least $10^2$ times higher than its specificity constant relative to the mono-galactosylated galactosyl acceptor.

Preferably, the specificity constant of the second enzyme relative to the free leaving group is:

- at least $10^3$ times higher than its specificity constant relative to the galactosyl donor,
- at least $10^3$ times higher than its specificity constant relative to the galactosyl acceptor, and
- at least $10^3$ times higher than its specificity constant relative to the mono-galactosylated galactosyl acceptor.

Alternatively, the specificity constant of the second enzyme relative to the free leaving group may be:

- at least $10^4$ times higher than its specificity constant relative to the galactosyl donor,
- at least $10^4$ times higher than its specificity constant relative to the galactosyl acceptor, and
- at least $10^4$ times higher than its specificity constant relative to the mono-galactosylated galactosyl acceptor.

The specificity constant of the second enzyme relative to the free leaving group may be:

- at least $10^5$ times higher than its specificity constant relative to the galactosyl donor,
- at least $10^5$ times higher than its specificity constant relative to the galactosyl acceptor, and
- at least $10^5$ times higher than its specificity constant relative to the mono-galactosylated galactosyl acceptor.

It is even more preferred that the specificity constant of the second enzyme relative to the free leaving group is:

- at least $10^6$ times higher than its specificity constant relative to the galactosyl donor,
- at least $10^6$ times higher than its specificity constant relative to the galactosyl acceptor, and
- at least $10^6$ times higher than its specificity constant relative to the mono-galactosylated galactosyl acceptor.

The specificity constant of the second enzyme relative to the free leaving group may for example be:

- at least $10^7$ times higher than its specificity constant relative to the galactosyl donor,
- at least $10^7$ times higher than its specificity constant relative to the galactosyl acceptor, and
- at least $10^7$ times higher than its specificity constant relative to the mono-galactosylated galactosyl acceptor.

It is particularly preferred that the second enzyme has glucose oxidase activity and the leaving group of the galactosyl donor is a glucosyl group in which case the free leaving group is glucose. If the second enzyme has glucose oxidase activity lactose is a preferred galactosyl donor.

Glucose oxidase require $O_2$ as oxidant and it may be necessary to add extra $O_2$ (g) to the incubating mixture if the normal amount of dissolved $O_2$ in water is insufficient. It is also possible to add co-factors such as FAD (flavin adenine dinucleotide) or NAD (nicotinamide adenine dinucleotide) to the incubating mixture if required.

As mentioned above, $H_2O_2$ is formed when glucose oxidase catalyses the oxidation of glucose to D-glucono-1,5-lactone, and high levels of $H_2O_2$ may be problematic for the process, e.g. due to undesired oxidation of the used enzymes. In some embodiments of the invention, the method furthermore involves providing a peroxidase or a catalase which contacts the incubating mixture and which catalyses the degradation of $H_2O_2$, e.g. to $H_2O$ and $O_2$. An example of a useful enzyme mixture which contains both glucose oxidase and catalase is Glyzyme BG (Novozymes, Denmark).

In some preferred embodiments of the invention, the method furthermore comprises providing a removal agent capable of removing at least some of the conversion product obtained by converting the leaving group with the second enzyme, and allowing the removal agent, during the incubation, to remove at least some of the conversion product.

In the context of the present invention the term "removal agent" pertains to a particulate or molecular agent capable of capturing conversion product and removing it from the incubating mixture, e.g. by precipitation.

The removal agent may for example comprise, or even consist of, a salt of a divalent or trivalent metal ion. Examples of useful metal ions are $Ca^{2+}$, $Fe^{2+}$, $Al^{3+}$, or $Zn^{2+}$.

In preferred embodiments of the invention, the removal agent comprises, or even consists of, $CaCO_3$.

Negatively charged conversion products such as e.g. gluconate, the deprotonated form of gluconic acid, may be removed using anion exchange chromatography.

Thus, the removal agent comprises, or even consists of, an anion exchange material.

When employed, the removal agent is preferably present in an amount sufficient to precipitate or capture the theoretical amount of converted leaving group formed during the synthesis process.

The inventors have discovered that the present method surprisingly provides a high yield of galacto-oligosaccharides even though a relatively low concentration of the galactosyl donor is used. The relatively low concentration of galactosyl donor additionally reduces the degree of self-galactosylation of the donor, i.e. when the galactosyl group of a first galactosyl donor is transferred to a second galactosyl donor instead of to a galactosyl acceptor.

In some preferred embodiments of the invention, step c) comprises addition of further galactosyl donor to the mixture. This is particularly preferred when a relatively low concentration of the galactosyl donor is used. By adding more galactosyl donor one avoids the galactosyl donor being depleted in the mixture and the concentration of galactosyl donor may be controlled during the enzymatic reaction.

The addition of further galactosyl donor may involve discrete addition(s) of galactosyl donor, e.g. at least once during the enzymatic reaction. Alternatively, or additionally, the addition of further galactosyl donor may be a continuous addition during the enzymatic reaction. The further galactosyl donor is preferably of the same type as used in step a).

Step c) may for example involve measuring the concentration of galactosyl donor during incubation and adding extra galactosyl donor if the concentration is too low.

In some preferred embodiments of the invention, the concentration of galactosyl donor of the mixture during step c) is maintained at a concentration in the range of 0.01-1 mol/L, preferably in the range of 0.01-0.5 mol/L, and preferably in the range of 0.03-0.3 mol/L.

For example, the concentration of galactosyl donor of the mixture during step c) may be maintained at a concentration in the range of 0.02-0.1 mol/L.

Step c) may furthermore comprise addition of further galactosyl acceptor. This makes it possible to control the concentration of galactosyl acceptor of the mixture during step c) and e.g. to keep the galactosyl acceptor concentration substantially constant if this is desired.

In order to produce significant amounts of galacto-oligosaccharides, which contain two or three transferred galactosyl groups, the process should consume more galactosyl donor than galactosyl acceptor. Thereby more of the galactosyl acceptors will become galactosylated two or three times. Thus, in some preferred embodiments of the invention the molar ratio between the consumed galactosyl donor and the consumed galactosyl acceptor is at least 1:1, and preferably at least 5: 1, and even more preferably at least 10:1.

Often it is required to enrich and/or purify the galacto-oligosaccharides of the composition and reduce the concentration of the galactosyl acceptor, the galactosyl donor and the released leaving group.

Thus, in some preferred embodiments of the invention the method furthermore comprises the step:

    d) enriching the galacto-oligosaccharides of the composition of step c).

In the context of the present invention, the term "enriching the galacto-oligosaccharides" relates to increasing the relative amount of the galacto-oligosaccharides of the composition on a dry weight basis. This is typically done by removing some of the other solids of the composition, e.g. the lower saccharides, and optionally also the first enzyme, if required.

The enrichment of step d) may for example involve chromatographic separation and/or nanofiltration. Details regarding such processes are described in Walstra *et al.* (2006) which is incorporated herein by reference for all purposes.

In some embodiments of the invention the enrichment involves that at least 50% (w/w on dry weight basis) of the molecules having a molar weight of at most 200 g/mol are removed from the composition of step c). For example, the enrichment may involve that at least 80% (w/w on dry weight basis) of the molecules having a molar weight of at most 200 g/mol are removed from the composition of step c).

In other embodiments of the invention the enrichment involves that at least 50% (w/w on dry weight basis) of the molecules having a molar weight of at most 350 g/mol are removed from the composition of step c). For example, the enrichment may involve that at least 80% (w/w on dry weight basis) of the molecules having a molar weight of at most 350 g/mol are removed from the composition of step c).

As an alternative, or in addition, to the enrichment it may be preferred that step d) comprises one or more processes which increase the concentration of the galacto-oligosaccharides in the composition. Examples of useful concentration steps are e.g. reverse osmosis, evaporation, and/or spray-drying.

Step d) may furthermore involve removing removal agent and/or converted leaving groups bound to the removal agent from the composition of step c). Such removal may e.g. be performed by filtration, sedimentation, or centrifugation.

The galacto-oligosaccharide-containing composition provided by the method may for example be in the form of a dry powder or in the form of a syrup.

The production of a dry powder typically requires one or more process steps, such as concentrating, evaporating, and/or spray-drying. Thus, in some preferred embodiments of the invention step d) furthermore involves concentrating, evaporating, and/or spray-drying the composition in liquid form to obtain the composition in powder form. It is particularly preferred to spray-dry the liquid composition of step d) to obtain a powdered composition. Step d) may for example comprise the enrichment step followed by concentration step, e.g. nanofiltration, reverse osmosis, or evaporation, followed by a spray-drying step. Alternatively, step d) may comprise the concentration step followed by an enrichment step, followed by a spray-drying step. Concentrating the galacto-oligosaccharides of the composition prior to the enrichment may make the subsequent enrichment process more cost-efficient.

Efficient spray-drying may require addition of one or more auxiliary agent(s), such as maltodextrin, milk protein, caseinate, whey protein concentrate, and/or skimmed-milk powder.

The present process may e.g. be implemented as a batch process. The present process may alternatively be implemented as a fed-batch process. The present process may alternatively be implemented as a continuous process.

The present process may furthermore involve recirculation of first enzyme and/or unused galactosyl acceptor back to the mixture. The recirculation may e.g. form part of step d). For example, step d) may involve separating galactosyl acceptor and/or the first enzyme from the galacto-oligosaccharide-containing composition and recirculating galactosyl acceptor and/or first enzyme to step a) or c). In the case of a batch process or a fed-batch process, the galactosyl acceptor and/or the first enzyme may be recirculated to the mixture of the next batch.

In the case of a continuous process, the galactosyl acceptor may be recirculated back to part of the process line corresponding to step a) or step c). The first enzyme may be recirculated back to part of the process line corresponding to step b) or step c).

It should be noted that the details and features related to steps a) and b) need not relate to the actual start of a production process but should at least occur sometime during the process. However, in some embodiments of the invention the concentration of the galactosyl donor is kept within the range described in step a) during the entire duration of step c).

If the method is implemented as a batch or feed batch process, step a) preferably pertains to the composition of the mixture when the synthesis starts. If the method is a continuous process, step a) preferably pertains to the composition of the mixture during the synthesis under steady-state operation.

It may be perceived as desirable that the level of galactosylated galactosyl donor is kept as low as possible, as galactosylated galactosyl donor may be perceived as an undesired impurity, which is tricky to separate from the galactosylated galactosyl acceptor. In some preferred embodiments of the invention, the incubating mixture of step c) contains at most 0.5 mol/L galactosylated galactosyl donor. The incubating mixture of step c) may for example contain at most 0.1 mol/L galactosylated galactosyl donor. Even more preferably the incubating mixture of step c) contains at most 0.01 mol/L galactosylated galactosyl donor, and preferably substantially no galactosylated galactosyl donor.

In some preferred embodiments of the invention where the donor is lactose, the total concentration of allo-lactose and galactosylated allo-lactose is kept as low as possible, as these compounds also are perceived as an undesired impurities, which is difficult to separate from the galactosylated galactosyl acceptor.

In some preferred embodiments of the invention, the incubating mixture of step c) contains a total amount of allo-lactose and galactosylated allo-lactose of at most 0.5 mol/L. The incubating mixture of step c) may for example contain a total amount of allo-lactose and galactosylated allo-lactose of at most 0.1 mol/L. Even more preferably the incubating mixture of step c) contains a total amount of allo-lactose and galactosylated allo-lactose of at most 0.01 mol/L, and preferably substantially no allo-lactose and galactosylated allo-lactose at all.

Yet an aspect of the invention relates to a composition comprising galacto-oligosaccharides, which composition is obtainable by the method as defined herein.

A further aspect of the invention is a galacto-oligosaccharide-containing composition, e.g. the above-mentioned composition, said galacto-oligosaccharide-containing composition comprising:

- a first galacto-oligosaccharide having the general formula Gal-X,
- a second galacto-oligosaccharide having the stoichiometric formula $(Gal)_2X$, such as e.g. the general formula Gal-Gal-X,
- a third galacto-oligosaccharide having the stoichiometric formula $(Gal)_3X$, such as e.g. the general formula Gal-Gal-Gal-X, and

wherein X is a glycosyl group, which is not lactosyl or glucosyl.

The galacto-oligosaccharide-containing composition described herein may for example be a food ingredient.

As described above, "X" or "-X" is preferably a glycosyl group of one of the galactosyl acceptors mentioned herein.

In some embodiments of the invention "X" or "-X" is a glycosyl group of a monosaccharide, which is not glucose. In other embodiments of the invention "-X" is a glycosyl group of a disaccharide, which is not lactose.

In some preferred embodiments of the invention "X" or "-X" is a fucosyl group. In other preferred embodiments of the invention "X" or "-X" is a galactosyl group.

In some preferred embodiments of the invention the galacto-oligosaccharide-containing composition has a molar ratio between:

- the total amount of the galacto-oligosaccharides Gal-X, $(Gal)_2X$, and $(Gal)_3X$, and
- the total amount of the galacto-oligosaccharides Gal-Glc, $Gal_2Glc$, $Gal_3Glc$

of at least 5:95. For example, the above-mentioned molar ratio may be at least 1:4, preferably at least 1:1, and even more preferably at least 2:1. It may even be preferred that the above-mentioned molar ratio is at least 5: 1, preferably at least 10: 1, and even more preferably at least 20: 1.

In other preferred embodiments of the invention the galacto-oligosaccharide-containing composition has a molar ratio between:

- the total amount of the galacto-oligosaccharides Gal-X, Gal-Gal-X, and Gal-Gal-Gal-X, and
- the total amount of the galacto-oligosaccharides Gal-Glc, Gal-Gal-Glc, Gal-Gal-Gal-Glc

of at least 5:95. For example, the above-mentioned molar ratio may be at least 1:4, preferably at least 1:1, and even more preferably at least 2:1. It may even be preferred that the above-mentioned molar ratio is at least 5: 1, preferably at least 10: 1, and even more preferably at least 20: 1.

It is even possible that the galacto-oligosaccharide-containing composition does not contain any galacto-oligosaccharides of the formula Gal-Glc, Gal-Gal-Glc, and Gal-Gal-Gal-Glc at all.

In some embodiments of the invention the galacto-oligosaccharide-containing composition has a molar ratio between the first galacto-oligosaccharide, the second galacto-oligosaccharide, and the third galacto-oligosaccharide in the range of 50-99 : 1-45 : 0.5-25.

In other embodiments of the invention the galacto-oligosaccharide-containing composition has a molar ratio between the first galacto-oligosaccharide, the second galacto-oligosaccharide, and the third galacto-oligosaccharide in the range of 20-45 : 20-45: 20-45.

In further embodiments of the invention the galacto-oligosaccharide-containing composition has a molar ratio between the first galacto-oligosaccharide, the second galacto-oligosaccharide, and the third galacto-oligosaccharide in the range of 0.5-25 : 1-45 : 50-98.

In some preferred embodiments of the invention the galacto-oligosaccharide-containing composition comprises a total amount of the first galacto-oligosaccharide, second galacto-oligosaccharide, and third galacto-oligosaccharide of at least 10% by weight relative to the total weight of the galacto-oligosaccharide-containing composition. For example, the galacto-oligosaccharide-containing composition may comprise a total amount of the first galacto-oligosaccharide, second galacto-oligosaccharide, and third galacto-oligosaccharide of at least 20% by weight relative to the total weight of the galacto-oligosaccharide-containing composition, preferably at least 30% by weight, even more preferably at least 40% relative to the total weight of the galacto-oligosaccharide-containing composition.

Even higher levels of the first, second, and third galacto-oligosaccharides may be preferred. Thus, in some preferred embodiments of the invention the galacto-oligosaccharide-containing composition comprises a total amount of the first galacto-oligosaccharide, second galacto-oligosaccharide, and third galacto-oligosaccharide of at least 50% by weight relative to the total weight of the galacto-oligosaccharide-containing composition. For example, the galacto-oligosaccharide-containing composition may comprise a total amount of the first galacto-oligosaccharide, second galacto-oligosaccharide, and third galacto-oligosaccharide of at least 60% by weight relative to the total weight of the galacto-oligosaccharide-containing composition, preferably least 70% by weight, even more preferably at least 80% relative to the total weight of the galacto-oligosaccharide-containing composition.

Yet an aspect of the invention relates to a food product comprising the galacto-oligosaccharide-containing composition described herein.

In some embodiments of the invention the food product is a functional food product such as infant formula or a product for clinical nutrition.

In other embodiments of the invention the food product is a baked product, e.g. comprising baked dough, such as bread or similar products.

In further embodiments of the invention the food product is a dairy product, e.g. a fresh dairy product such as milk, or a fermented dairy product such as yoghurt.

In still further embodiments of the invention the food product is a pet food product.

It should be noted that embodiments and features described in the context of one of the aspects of the present invention also apply to the other aspects of the invention.

The invention will now be described in further details in the following non-limiting examples.

## EXAMPLES

Example 1: Preparation of a beta-aalactosidase having transgalactosylating activity

A working volume of 750 mL fermentation medium was inoculated with a 2 mL starter-culture of Lysogeny broth (LB) medium with 100 mg/L ampicillin with an $OD_{600}$ of 3.0 grown for 12 hours. The fermentation was performed in EC medium containing 2 % (w/v) yeast extract, 2 % (w/v) soy peptone, 1 % (w/v) glucose and 100 mg/L ampicillin. The *E. coli* strain expressing OLGA347 β-galactosidase was prepared as described earlier (Jørgensen et al., US patent No.

6,555,348 B2, Examples 1 and 2). The fermentor was from Applikon with glass dished bottom vessels with a total volume of 2 L and equipped with two Rushton impellers. During the fermentation, pH was maintained at pH 6.5 by appropriate addition of 2 M NaOH and 2 M $H_3PO_4$ and temperature was controlled at 37 degrees C. Oxygen was supplied by bubbling with air at a rate of 1-2 L/min, and $pO_2$ was maintained at 30% by increasing the agitation rate. Growth was followed by offline $OD_{600}$ readings. The culture was harvested by centrifugation after approximately 10 h of growth at an $OD_{600}$ value of 29.7. The 650 mL culture supernatant was stored at -20 degrees C. The periplasmic proteins were isolated from the cell pellet by osmotic shock by resuspending the cell pellet in 200 mL sucrose buffer (30 mM Tris-HCl, 40% sucrose, 2 mM EDTA, pH 7.5) and incubating for 10 min at room temperature. After centrifugation, the supernatant was discarded and the pellet resuspended in 200 mL of cold water. 83 $\mu$L of a saturated $MgCl_2$ solution was added, and the supernatant containing the periplasmic proteins were collected by a centrifugation step. The periplasmic fraction was filter sterilized through a 0.2 $\mu$m Millipak 40 filter and stored at -20 degrees C.

The $\beta$-galactosidase activity of the 200 mL periplasmic fraction and the 650 mL culture supernatant was determined using o-nitrophenyl-$\beta$-D-galactopyranoside (OPNG) as a substrate according to protocol (J. Sambrook and D.W. Russell, Molecular Cloning - A laboratory manual, 3rd edition (2001), pp. 17.48-17.51). The majority of the activity was found in the periplasmic fraction (525 units, corresponding to 98%).

Example 2: Determination of the T-value of a beta-galactosidase enzyme

The T-value of a beta-galactosidase enzyme is determined according to the assay and formula given below.

Assay:

Prepare 3.3 mL enzyme solution consisting of the beta-galactosidase enzyme to be tested, 10 mM sodium citrate, 1 mM magnesium citrate, 1 mM calcium-citrate, Milli-Q water (Millipore, USA), and having a pH of 6.5. The enzyme solution should contain the beta-galactosidase enzyme in an amount sufficient to use 33% (w/w) of the added lactose in 1 hour under the present assay condition. The temperature of the enzyme solution should be 37 degrees C.

At time = To 82.5 mg lactose monohydrate (for biochemistry, Merck Germany) is added to and mixed with the enzyme solution, and the mixture is subsequently incubated at 37 degrees C for 4 hours. Precisely 1 hour after To a 100 $\mu$L sample is collected and is diluted 1:5 with Milli-Q water and inactivated by heating to 85 °C for 10 min. The inactivated mixture is kept at -20 degrees C until the characterization.

Characterisation:

The determination of the amount (in mol) of produced galactose and the amount of used lactose (in mol) may be performed using any suitable analysis technique. For example, the diluted mixture may be analyzed by HPLC according to the method described by Richmond *et al.* (1982) and Simms *et al.* (1994). Other useful analysis techniques are described in El Razzi (2002).

Another example of a suitable analysis technique is ISO 5765-2:2002 (IDF 79-2: 2002) "Dried milk, dried ice-mixes and processed cheese - Determination of lactose content - Part 2: Enzymatic method utilizing the galactose moiety of the lactose".

Calculation of the T-value:

The T-value is calculated according to the following formula using the data obtained from the characterization of the diluted mixture of the assay:

$$\text{T-value} = \frac{\text{amount of produced galactose (in mol)}}{\text{amount of used lactose (in mol)}}$$

Example - T-value of the OLGA347 enzyme:

The above-mentioned assay was performed using the OLGA347 enzyme of Example 1.

The diluted mixture obtained from the assay was analyzed with respect to converted (i.e. used) lactose and generated galactose via analytical HPLC. The HPLC apparatus was from Waters and equipped with a differential refractometer (RI-detector) and a BioRad Aminex HPX-87C column (300x7.8 mm, 125-0055). Elution of saccharides was performed isocratically with 0.05 g/L CaAcetate, a flow rate of 0.3 mL/min. and an injection volume of 20 $\mu$L.

The obtained data was appropriately baseline corrected by automated software, peaks were individually identified and integrated. Quantification was performed by using external standards of lactose monohydrate (for biochemistry, Merck, Germany), D-(+)-glucose monohydrate (for biochemistry, Merck Eurolab, France), and D-(+)-galactose (≥99%, Sigma-Aldrich, Italy).

The conversion of lactose and the formation of galactose to each time T was calculated from the quantified data. At time T=1h 29% of the lactose in the collected 100 μL sample had been converted, which corresponds to 2.3 μmol lactose. At time T=1 0.5 μmol galactose had been formed in the collected 100 μL sample. The T-value can therefore be calculated to 0.5 μmol / 2.3 μmol = 0.2.

The diluted mixture obtained from the assay was also analyzed with respect to converted (i.e. used) lactose and generated galactose via the enzymatic method ISO 5765-2. A Boehringer Mannheim Lactose/D-Galactose test-kit from R-Biopharm (Cat. No. 10 176 303 035) was used and the test performed according to protocol. The enzymatic method confirmed a T-value of the OLGA347-enzyme of 0.2.

Example - T-value of conventional lactase enzyme:

The above-mentioned assay was performed using the commercially available conventional lactase enzyme Lactozym Pure 2600L (Novozymes, Denmark). The diluted mixture obtained from the assay was analyzed as described for the OLGA347 enzyme. Tri- and tetra-saccharides were not present in detectable amounts and equal amounts of glucose and galactose were seen. The corresponding T-value is 1.

The T-values of commercially available beta-galactosidase from *Escherichia coli* (Product number: G6008, Sigma-Aldrich, Germany) and *Aspergillus oryzae* (Product number: G5160, Sigma-Aldrich, Germany) have also been determined, and both enzymes have a T-value of approx. 1.

Example 3: Synthesis of L-fucosyl-containing hetero-galacto-oligosaccharides by sequential addition of donor molecules and conversion of glucose leaving groups

L-(-)-Fucose and lactose monohydrate in amounts as found in Table 6 is dissolved in 500 mL MilliQ water. Each sample is maintained at a temperature of 37 degrees C and pH is maintained at 6.5 by continuous addition of NaOH. OLGA347 enzyme prepared as in Example 1 and Glucose Oxidase enzyme, GOX, Danisco, Denmark, is added to each sample. This time is defined as T=0. Lactose monohydrate is added to the samples over a 10 hour period in 30 min. intervals. At times T=0, 1, 2, 3, 4, 5, 6, 7, 8, 9 and 10 h 100 μL samples are acquired and the amount of produced L-fucosyl-containing hetero-galacto-oligosaccharides is determined as in Example 7.

Table 6:

| Sample | 1 | 2 | 3 | 4 |
|---|---|---|---|---|
| **Start concentration of L-Fucose** | 10 % | 10 % | 20 % | 20 % |
| **Start concentration of lactose monohydrate** | 5 % | 5% | 5% | 5% |
| **Addition of lactose / 30 min.** | 1,12 g | 2,23 g | 1,12 g | 2,23 g |
| **Amount of Glucose oxidase** | 6,9 mg | 13,8 mg | 6,9 mg | 13,8 mg |
| **Specific activity of Glucose oxidase** | 11.000 U/g | 11.000 U/g | 11.000 U/g | 11.000 U/g |
| **Amount of OLGA enzyme** | 16,28 mL | 16,28 mL | 16,28 mL | 16,28 mL |

Example 4: Synthesis of L-fucosyl-containing hetero-galacto-oligosaccharides and conversion of glucose leaving groups but without sequential addition of donor molecules

L-(-)-Fucose and lactose monohydrate in amounts as found in Table 7 is dissolved in 500 mL MilliQ water. Each sample is maintained at a temperature of 37 degrees C and pH is maintained at 6.5 by continuous addition of NaOH. OLGA347 enzyme prepared in Example 1 and Glucose Oxidase enzyme, GOX, Danisco, Denmark, is added to each sample. This time is defined as T=0. At times T=0, 1, 2, 3, 4, 5, 6, 7, 8, 9 and 10 h 100 μL samples are acquired and the amount of produced L-fucosyl-containing hetero-galacto-oligosaccharides is determined as in Example 7.

Table 7:

| Sample | 1 | 2 |
|---|---|---|
| **Start concentration of L-Fucose** | 20 % | 20 % |

(continued)

| Sample | 1 | 2 |
|---|---|---|
| **Start concentration of lactose monohydrate** | 20 % | 20 % |
| **Amount of Glucose oxidase** | 6,9 mg | 13,8 mg |
| **Specific activity of Glucose oxidase** | 11.000 U/g | 11.000 U/g |
| **Amount of OLGA enzyme** | 16,28 mL | 16,28 mL |

Example 5: Synthesis of L-fucosyl-containing hetero-galacto-oligosaccharides by sequential addition of donor molecules without conversion of glucose leaving groups

L-(-)-Fucose and lactose monohydrate in amounts as found in Table 8 is dissolved in 500 mL MilliQ water. Each sample is maintained at a temperature of 37 degrees C and pH is maintained at 6.5 by continuous addition of NaOH. OLGA347 enzyme prepared as in Example 1 is added to each sample. This time is defined as T=0. Lactose monohydrate is added to the samples over a 10 hour period in 30 min. intervals. At times T=0, 1, 2, 3, 4, 5, 6, 7, 8, 9 and 10 h 100 $\mu$L samples are acquired and the amount of produced L-fucosyl-containing hetero-galacto-oligosaccharides is determined as in Example 7.

Table 8:

| Sample | 1 | 2 | 3 | 4 |
|---|---|---|---|---|
| **Start concentration of L-Fucose** | 10 % | 10 % | 20 % | 20 % |
| **Start concentration of lactose monohydrate** | 5 % | 5% | 5% | 5% |
| **Addition of lactose / 30 min.** | 1,12 g | 2,23 g | 1,12 g | 2,23 g |
| **Amount of OLGA enzyme** | 16,28 mL | 16,28 mL | 16,28 mL | 16,28 mL |

Example 6: Synthesis of L-fucosyl-containing hetero-galacto-oligosaccharides without conversion of glucose leaving groups and without sequential addition of donor molecules

L-(-)-Fucose and lactose monohydrate in amounts as found in Table 9 is dissolved in 500 mL MilliQ water. Each sample is maintained at a temperature of 37 degrees C and pH is maintained at 6.5 by continuous addition of NaOH. OLGA347 enzyme prepared as in Example 1 is added to each sample. This time is defined as T=0. At times T=0, 1, 2, 3, 4, 5, 6, 7, 8, 9 and 10 h 100 $\mu$L samples are acquired and the amount of produced L-fucosyl-containing hetero-galacto-oligosaccharides is determined as in Example 7.

Table 9:

| Sample | 1 |
|---|---|
| **Start concentration of L-Fucose** | 20 % |
| **Start concentration of lactose monohydrate** | 20 % |
| **Amount of OLGA enzyme** | 16,28 mL |

Example 7: Determination of amount of produced L-fucosyl-containing hetero-galacto-oligosaccharides

Collected samples are diluted 1:5 with Milli-Q water and inactivated by heating to 85 °C for 10 min. The inactivated mixture is kept at -20 degrees C until the characterization.

Samples are characterized by use of analytical HPLC. The HPLC apparatus is from Waters and equipped with a differential refractometer (RI-detector) and a BioRad Aminex HPX-87C column (300x7.8 mm, 125-0055). Elution of saccharides is performed isocratically with 0.05 g/L CaAcetate, a flow rate of 0.3 mL/min. and an injection volume of 20 $\mu$L.

Mass spectrometry analysis is performed with an Agilent 1100 API-ES LC/MSD Quadropole scanning masses between 100 and 1000 amu (gas temperature: 350 °C, drying gas flow: 13.0 L/min, nebulizer pressure: 60 psig). The detected ions arise from complexation between the analyte and sodium cations from the solution, resulting in detected masses of $M(Na^+) = M + 23$ Da. The obtained data is appropriately baseline corrected by automated software, peaks are individually identified and integrated. The amount of produced saccharides in mols are calculated by comparing the peaks of glucose, galactose, hetero-galactose-oligosaccharides (Gal-Fuc, Gal-Gal-Fuc, Gal-Gal-Gal-Fuc), and galacto-oligosaccharides (Gal-Gal-Glc, Gal-Gal-Gal-Glc) to synthesized standards.

**REFERENCES**

Buchholz (2005)          "Biocatalysts and Enzyme technology", Klaus Buchholz et al., ISBN-10: 3-527-30497-5, 2005, Wiley VCH Verlag GmbH

Franck (2002)            "Technological functionality of inulin and oligofructose", A. Franck, British Journal of Nutrition (2002), 87, Suppl. 2, S287-S291

Yun (1996)               "Fructooligosaccharides-Occurrence, preparation, and application", J. W. Yun, Enzyme and Microbial Technology 19: 107-117, 1996

Kunz (2000)              "Oligosaccharides in human milk: Structural, functional and metabolic aspects", Kunz et al., Ann. Rev. Nutr. 2000. 20:699-722

Simms *et al.* (1994)    Simms, P.J.; Hicks, K.B.; Haines, R.M.; Hotchkiss, A.T. and Osman, S.F.; (1994) Separations of lactose, lactobionic and lactobionolactose by high performance liquid chromatography. J. of Chromatography, 667, 67-73.

Richmond *et al.* (1982) Richmond, M.L.; Barfuss, D.L.; Harte, B.R.; Gray, J.I. and Stine, C.M.; (1982) Separation of Carbohydrates in Dairy Products by High Performance Liquid Chromatography, J. of Dairy Science, 65 (8), 1394-1400.

El Razzi (2002)          "Carbohydrate Analysis by Modern Chromatography and Electrophoresis", volume 66, Journal of Chromatography Library, Elsevier Science, 2002, ISBN-10: 0444500618

Walstra *et al.* (2006)  "Dairy science and technology", Walstra et al., CRC Press, Second edition, 2006

**Claims**

1. A method of producing a composition comprising one or more galacto-oligosaccharide(s), the method comprising the steps of:

   a) providing a mixture comprising

   - a galactosyl donor comprising a galactosyl group bound to a leaving group, which galactosyl donor has a molar weight of at most 350 g/mol,
   - a galactosyl acceptor which is different from the galactosyl donor,
   said galactosyl acceptor is a saccharide or a sugar-alcohol, and
   wherein the molar ratio between the galactosyl acceptor and the galactosyl donor is at least 1:10, and
   wherein the mixture comprises at least 0.05 mol/L of the galactosyl acceptor,

   b) providing a first enzyme, said first enzyme having beta-galactosidase activity and a T-value of at most 0.9, said first enzymes contacting the mixture, and
   c) incubating the mixture and the first enzyme, thereby allowing the first enzyme to release the leaving group of the galactosyl donor and transfer the galactosyl group of the galactosyl donor to the galactosyl acceptor, thus forming the galacto-oligosaccharide, step c) furthermore comprising removing from the incubating mixture a leaving group released from the galactosyl donor, thereby obtaining the composition comprising the one or more galacto-oligosaccharide(s).

2. The method according to claim 1, wherein the leaving group of the galactosyl donor is a glycosyl group.

3. The method according to any of the preceding claims, wherein the first enzyme comprises:

   - an amino acid sequence having a sequence identity of at least 80% relative to the amino acid sequence of SEQ ID NO. 2, or
   - an amino acid sequence having a sequence identity of at least 80% relative to the amino acid sequence Met (1) to Ile (1174) of SEQ ID NO. 2, or
   - an amino acid sequence having a sequence identity of at least 80% relative to the amino acid sequence Val (33) to Gly (950) of SEQ ID NO. 2, or
   - an amino acid sequence having a sequence identity of at least 80% relative to the amino acid sequence Val (33) to Ile (1174) of SEQ ID NO. 2.

4. The method according to any of the preceding claims, furthermore comprising providing a microorganism which is capable of converting free leaving groups released from the galactosyl donor, and allowing said microorganism, during incubation, to remove a leaving group released from the galactosyl donor.

5. The method according to claim 4, wherein

   a) the removal rate of the microorganism relative to the free leaving group is at least 10 times higher than its removal rate relative to the galactosyl acceptor,
   b) the removal rate of the microorganism relative to the free leaving group is at least 10 times higher than its removal rate relative to the galactosyl donor, or
   c) the removal rate of the microorganism relative to the free leaving group is at least 10 times higher than its removal rate relative to mono-galactosylated galactosyl acceptor.

6. The method according to any of the preceding claims, furthermore comprising providing a second enzyme which is capable of converting free leaving groups released from the galactosyl donor, and allowing said second enzyme, during incubation, to convert a leaving group released from the galactosyl donor.

7. The method according to claim 6, wherein the second enzyme has glucose oxidase activity.

8. The method according to any of the claims 6-7, wherein

   a) the specificity constant of the second enzyme relative to the free leaving group is at least 10 times higher than its specificity constant relative to the galactosyl acceptor,

b) the specificity constant of the second enzyme relative to the free leaving group is at least 10 times higher than its specificity constant relative to the galactosyl donor, or

c) the specificity constant of the second enzyme relative to the free leaving group is at least 10 times higher than its specificity constant relative to the mono-galactosylated galactosyl acceptor.

9. The method according to any of the claims 6-8, wherein the second enzyme has glucose oxidase activity and the leaving group of the galactosyl donor is glucose.

10. The method according to any of the claims 6-9, wherein the method furthermore comprises providing a removal agent capable of removing at least some of the conversion product obtained by converting the leaving group with the second enzyme, and allowing the removal agent, during the incubation, to remove at least some of the conversion product.

11. The method according to claim 10, wherein the removal agent comprises a salt of a divalent or trivalent metal ion.

12. The method according to any of the preceding claims, wherein step c) comprises addition of further galactosyl donor.

13. The method according to any of the preceding claims, wherein the concentration of galactosyl donor of the mixture during step c) is maintained at a concentration in the range of 0.01-1 mol/L.

14. The method according to any of the preceding claims furthermore comprising the step:

d) enriching the galacto-oligosaccharide of the composition of step c).

15. The method according to claim 14, wherein the step d) furthermore involves removing removal agent and/or converted leaving groups bound to the removal agent from the composition of step c).

# Fig. 1

Donor

First enzyme

a) + △

b) + [Gal]—[Glc]

c) + [Glc]

[Gal]—[Gal]—[Glc] +

[Gal] : Gal    [Glc] : Glc    △ : Acceptor    [Gal]~[Glc] : Allo-lactose

# Fig. 2

# Fig. 3

**EP 2 620 506 A1**

Europäisches Patentamt
European Patent Office
Office européen des brevets

# EUROPEAN SEARCH REPORT

Application Number

EP 12 15 2502

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 138 586 A1 (COGNIS IP MAN GMBH [DE]; UNIV MADRID COMPLUTENSE [ES]) 30 December 2009 (2009-12-30) * paragraphs [0009], [0014], [0016], [0019]; claims 3, 4, 7, 10 * ----- | 1 | INV. C12P19/00 C12P19/04 C12P19/16 C12P19/20 |
| A | WO 2009/113030 A2 (TATA CHEMICALS LTD [IN]; AVALAKKI UDAY KASHINATH [IN]; MAHESWARAN PALA) 17 September 2009 (2009-09-17) ----- | 1 | |
| E | WO 2012/010597 A1 (ARLA FOODS AMBA [DK]; BERTELSEN HANS [DK]; LANGBORG WEJSE PETER [DK]) 26 January 2012 (2012-01-26) * page 25, line 30 - page 26, line 6 * ----- | 1 | |
| | | | TECHNICAL FIELDS SEARCHED (IPC) C12P |

The present search report has been drawn up for all claims

| Place of search | Date of completion of the search | Examiner |
|---|---|---|
| Berlin | 7 June 2012 | Panzica, Gian Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

49

**ANNEX TO THE EUROPEAN SEARCH REPORT
ON EUROPEAN PATENT APPLICATION NO.**

EP 12 15 2502

This annex lists the patent family members relating to the patent documents cited in  the above-mentioned European search report.
The members are as contained in the European Patent Office EDP file on
The European Patent Office is in no way liable for these particulars which are merely given for the purpose of information.

07-06-2012

| Patent document cited in search report | | Publication date | Patent family member(s) | | Publication date |
|---|---|---|---|---|---|
| EP 2138586 | A1 | 30-12-2009 | NONE | | |
| WO 2009113030 | A2 | 17-09-2009 | EP | 2252699 A2 | 24-11-2010 |
| | | | JP | 2011517553 A | 16-06-2011 |
| | | | US | 2011065152 A1 | 17-03-2011 |
| | | | WO | 2009113030 A2 | 17-09-2009 |
| WO 2012010597 | A1 | 26-01-2012 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0190317 A2
- WO 2011120993 A1

- US 6555348 B2, Jørgensen

### Non-patent literature cited in the description

- **J. SAMBROOK ; D.W. RUSSELL.** Molecular Cloning - A laboratory manual. 2001, 17.48-17.51
- **KLAUS BUCHHOLZ et al.** Biocatalysts and Enzyme technology. Wiley VCH Verlag, 2005
- **A. FRANCK.** Technological functionality of inulin and oligofructose. *British Journal of Nutrition,* 2002, vol. 87 (2), S287-S291
- **J. W. YUN.** Fructooligosaccharides-Occurrence, preparation, and application. *Enzyme and Microbial Technology,* 1996, vol. 19, 107-117
- **KUNZ et al.** Oligosaccharides in human milk: Structural, functional and metabolic aspects. *Ann. Rev. Nutr.,* 2000, vol. 20, 699-722

- **SIMMS, P.J. ; HICKS, K.B. ; HAINES, R.M. ; HOTCHKISS, A.T. ; OSMAN, S.F.** Separations of lactose, lactobionic and lactobionolactose by high performance liquid chromatography. *J. of Chromatography,* 1994, vol. 667, 67-73
- **RICHMOND, M.L. ; BARFUSS, D.L. ; HARTE, B.R. ; GRAY, J.I. ; STINE, C.M.** Separation of Carbohydrates in Dairy Products by High Performance Liquid Chromatography. *J. of Dairy Science,* 1982, vol. 65 (8), 1394-1400
- Carbohydrate Analysis by Modern Chromatography and Electrophoresis. Journal of Chromatography Library. Elsevier Science, 2002, vol. 66
- **WALSTRA et al.** Dairy science and technology. CRC Press, 2006

SEQUENCE LISTING

<110>  Arla Foods amba

<120>  Method of producing a galacto-oligosaccharide-containing composition

<130>  P1120EP00

<160>  2

<170>  PatentIn version 3.1

<210>  1
<211>  5509
<212>  DNA
<213>  Bifidobacterium bifidum

<220>
<221>  CDS
<222>  (212)..(5470)
<223>

<400>  1
```
atgcgttgcg ttgcgatttt tccggccctg tatgggggat acaggattgg cgatggcgac       60

acgccgtttt tgttaatggc atttacatga aatacaggta atgagatatc attctcatga      120

tcaccgtgtg gatatcgcat tggtgcgtat acactaacag caacagagcg gcgcggcagg      180

cgctcgtgga ttcaatgaag aaggaacgtt t atg gca gtt cgc aga ctt ggt         232
                                    Met Ala Val Arg Arg Leu Gly
                                    1               5

ggc cgc atc gtg gct ttc gcc gcc aca gtg gcc ttg tca ata ccg tta        280
Gly Arg Ile Val Ala Phe Ala Ala Thr Val Ala Leu Ser Ile Pro Leu
        10               15               20

ggg ttg tta aca aat tca gcg tgg gcg gtc gag gac gcc acc cga tcc        328
Gly Leu Leu Thr Asn Ser Ala Trp Ala Val Glu Asp Ala Thr Arg Ser
        25               30               35

gac tcc acc acg cag atg agc tcc acg ccg gag gtg gtc tac tcc agc        376
Asp Ser Thr Thr Gln Met Ser Ser Thr Pro Glu Val Val Tyr Ser Ser
40               45               50               55

gcc gtg gat tcc aag cag aat cgc acc tcg gat ttc gac gcc aac tgg        424
Ala Val Asp Ser Lys Gln Asn Arg Thr Ser Asp Phe Asp Ala Asn Trp
```

```
                  60                    65                    70
aag ttc atg ctg tcc gat tcc gtg cag gcg cag gat ccg gcg ttc gac      472
Lys Phe Met Leu Ser Asp Ser Val Gln Ala Gln Asp Pro Ala Phe Asp
        75                    80                    85

gat tcg gcc tgg cag cag gtc gac ctg ccg cat gac tac agc atc acg      520
Asp Ser Ala Trp Gln Gln Val Asp Leu Pro His Asp Tyr Ser Ile Thr
        90                    95                   100

cag aag tat tcg cag agc aac gag gcc gaa agc gca tac ctt ccc ggc      568
Gln Lys Tyr Ser Gln Ser Asn Glu Ala Glu Ser Ala Tyr Leu Pro Gly
       105                   110                   115

ggc acc ggc tgg tac cgc aag tcc ttc acc atc gac cgg gac ctc gcc      616
Gly Thr Gly Trp Tyr Arg Lys Ser Phe Thr Ile Asp Arg Asp Leu Ala
120                   125                   130                   135

ggc aag cgc atc gcc atc aac ttc gac ggc gtg tac atg aac gcc acc      664
Gly Lys Arg Ile Ala Ile Asn Phe Asp Gly Val Tyr Met Asn Ala Thr
                140                   145                   150

gtc tgg ttc aac ggc gtc aag ctc ggc acc cat ccg tac ggc tac tcg      712
Val Trp Phe Asn Gly Val Lys Leu Gly Thr His Pro Tyr Gly Tyr Ser
                155                   160                   165

ccg ttc tcc ttc gac ctg acc ggc aac gcc aag ttc ggt ggg gag aac      760
Pro Phe Ser Phe Asp Leu Thr Gly Asn Ala Lys Phe Gly Gly Glu Asn
        170                   175                   180

acc atc gtc gtc aag gtc gag aac agg ctg ccg tcc agc cgc tgg tac      808
Thr Ile Val Val Lys Val Glu Asn Arg Leu Pro Ser Ser Arg Trp Tyr
        185                   190                   195

tcc ggc tcc ggc atc tac cgc gac gtc acc ctc acc gtc acc gac ggc      856
Ser Gly Ser Gly Ile Tyr Arg Asp Val Thr Leu Thr Val Thr Asp Gly
200                   205                   210                   215

gtg cac gtc ggc aat aac ggc gtg gcc atc aag acc ccg agc ctc gcc      904
Val His Val Gly Asn Asn Gly Val Ala Ile Lys Thr Pro Ser Leu Ala
                220                   225                   230

acc caa aac ggc ggc gac gtg acg atg aac ctc acc acc aag gtc gcc      952
Thr Gln Asn Gly Gly Asp Val Thr Met Asn Leu Thr Thr Lys Val Ala
        235                   240                   245

aac gac acc gag gcc gcg gcg aac atc acc ctc aag cag acc gtg ttc     1000
```

```
Asn Asp Thr Glu Ala Ala Ala Asn Ile Thr Leu Lys Gln Thr Val Phe
    250                 255                 260

ccc aag gga ggc aag acc gac gcc gcc atc ggc acc gtc acc acc gca    1048
Pro Lys Gly Gly Lys Thr Asp Ala Ala Ile Gly Thr Val Thr Thr Ala
    265                 270                 275

tcc aag tcc atc gcg gcc ggt gcc agc gcg gac gtg acc tcc acg atc    1096
Ser Lys Ser Ile Ala Ala Gly Ala Ser Ala Asp Val Thr Ser Thr Ile
280                 285                 290                 295

acc gcc gct tcg ccc aag ctg tgg agc atc aag aac ccg aac ctg tac    1144
Thr Ala Ala Ser Pro Lys Leu Trp Ser Ile Lys Asn Pro Asn Leu Tyr
                300                 305                 310

acc gtg cgc acc gaa gtg ctc aac ggc ggc aag gtg ctc gac act tac    1192
Thr Val Arg Thr Glu Val Leu Asn Gly Gly Lys Val Leu Asp Thr Tyr
                315                 320                 325

gac acc gaa tat ggc ttc cgc tgg acc ggc ttc gat gcg acc agc ggt    1240
Asp Thr Glu Tyr Gly Phe Arg Trp Thr Gly Phe Asp Ala Thr Ser Gly
                330                 335                 340

ttc tcg ctc aac ggc gag aaa gtc aag ctc aag ggc gtc tca atg cat    1288
Phe Ser Leu Asn Gly Glu Lys Val Lys Leu Lys Gly Val Ser Met His
    345                 350                 355

cat gac cag gga tcg ctc ggc gcg gtc gcc aac cgc cgc gcc atc gag    1336
His Asp Gln Gly Ser Leu Gly Ala Val Ala Asn Arg Arg Ala Ile Glu
360                 365                 370                 375

cgc cag gtc gag att ctc cag aag atg ggc gtc aac tcg atc cgc acc    1384
Arg Gln Val Glu Ile Leu Gln Lys Met Gly Val Asn Ser Ile Arg Thr
                380                 385                 390

acg cac aac ccc gca gcc aag gcg ctg att gac gtc tgc aac gag aag    1432
Thr His Asn Pro Ala Ala Lys Ala Leu Ile Asp Val Cys Asn Glu Lys
                395                 400                 405

ggc gtc ctc gtg gtc gaa gag gtc ttc gac atg tgg aac cgg tcg aag    1480
Gly Val Leu Val Val Glu Glu Val Phe Asp Met Trp Asn Arg Ser Lys
                410                 415                 420

aac ggc aac acc gag gat tac ggc aag tgg ttc ggc cag gcc atc gcc    1528
Asn Gly Asn Thr Glu Asp Tyr Gly Lys Trp Phe Gly Gln Ala Ile Ala
    425                 430                 435
```

```
ggt gac aac gcc gtc ctg ggt ggc gac aag gac gag acc tgg gcc aag    1576
Gly Asp Asn Ala Val Leu Gly Gly Asp Lys Asp Glu Thr Trp Ala Lys
440             445             450             455

ttc gac ctg acc agc acc atc aac cgt gac agg aac gcc ccg tcc gtc    1624
Phe Asp Leu Thr Ser Thr Ile Asn Arg Asp Arg Asn Ala Pro Ser Val
            460             465             470

atc atg tgg tcg ctc ggc aac gag atg atg gaa ggc atc agc ggc agc    1672
Ile Met Trp Ser Leu Gly Asn Glu Met Met Glu Gly Ile Ser Gly Ser
        475             480             485

gtc tcg ggc ttc ccg gct acc tcc gcc aag ctg gtc gca tgg acg aag    1720
Val Ser Gly Phe Pro Ala Thr Ser Ala Lys Leu Val Ala Trp Thr Lys
        490             495             500

gcc gcg gac agc acc cgc ccg atg acc tac ggc gac aac aag atc aag    1768
Ala Ala Asp Ser Thr Arg Pro Met Thr Tyr Gly Asp Asn Lys Ile Lys
        505             510             515

gcc aac tgg aac gag tcg aac acc atg ggc gac aac ctg acc gcc aac    1816
Ala Asn Trp Asn Glu Ser Asn Thr Met Gly Asp Asn Leu Thr Ala Asn
520             525             530             535

ggc ggc gtg gtc ggc acc aac tac tcc gac ggc gcg aac tac gac aag    1864
Gly Gly Val Val Gly Thr Asn Tyr Ser Asp Gly Ala Asn Tyr Asp Lys
        540             545             550

atc cgc acg acc cac ccc tca tgg gcc atc tat ggt tcc gag acg gcg    1912
Ile Arg Thr Thr His Pro Ser Trp Ala Ile Tyr Gly Ser Glu Thr Ala
        555             560             565

tcc gcc atc aac agc cga ggc atc tac aac cgc acc acc ggc ggc gcc    1960
Ser Ala Ile Asn Ser Arg Gly Ile Tyr Asn Arg Thr Thr Gly Gly Ala
        570             575             580

cag tca agc gac aag cag ctg acc agc tat gac aat tcc gca gtc ggc    2008
Gln Ser Ser Asp Lys Gln Leu Thr Ser Tyr Asp Asn Ser Ala Val Gly
        585             590             595

tgg ggc gcc gtc gcc agc tcc gcc tgg tac gac gtg gtc cag cgc gat    2056
Trp Gly Ala Val Ala Ser Ser Ala Trp Tyr Asp Val Val Gln Arg Asp
600             605             610             615

ttc gtc gcc ggc aca tac gtg tgg acc ggc ttc gac tac ctc ggc gaa    2104
Phe Val Ala Gly Thr Tyr Val Trp Thr Gly Phe Asp Tyr Leu Gly Glu
        620             625             630
```

27

```
ccc acc ccg tgg aac ggc acc ggc tcc ggc gcc gtg ggc tcc ttg gcc      2152
Pro Thr Pro Trp Asn Gly Thr Gly Ser Gly Ala Val Gly Ser Leu Ala
            635                 640                 645

gtc gcc gaa gaa ctc gta ctt cgg cat cgt cga cac cgc agg ctt ccc      2200
Val Ala Glu Glu Leu Val Leu Arg His Arg Arg His Arg Arg Leu Pro
            650                 655                 660

gaa gac acc tat tac ttc tat cag agc cag tgg aac gac gac gtg cac      2248
Glu Asp Thr Tyr Tyr Phe Tyr Gln Ser Gln Trp Asn Asp Asp Val His
            665                 670                 675

acg ctg cac atc ctc ccc gca tgg aac gag aac gtc gtc gcc aag ggc      2296
Thr Leu His Ile Leu Pro Ala Trp Asn Glu Asn Val Val Ala Lys Gly
680                 685                 690                 695

tcc ggc aac aac gtg ccg gtc gtc gtc tac acc gac gcg gcc aag gtc      2344
Ser Gly Asn Asn Val Pro Val Val Val Tyr Thr Asp Ala Ala Lys Val
                700                 705                 710

aag ctg tac ttc aca ccg aag ggc agt acc gaa aag cga ctg atc gga      2392
Lys Leu Tyr Phe Thr Pro Lys Gly Ser Thr Glu Lys Arg Leu Ile Gly
                715                 720                 725

gag aag tcc ttc acc aag aag acc acc gcg gcc gga tac acc tat cag      2440
Glu Lys Ser Phe Thr Lys Lys Thr Thr Ala Ala Gly Tyr Thr Tyr Gln
            730                 735                 740

gtc tac gag ggc tcc gac aag gac tcc acc gcc cac aag aac atg tac      2488
Val Tyr Glu Gly Ser Asp Lys Asp Ser Thr Ala His Lys Asn Met Tyr
            745                 750                 755

ctg acc tgg aac gtg ccg tgg gcc gag ggc acc atc tcc gcc gaa gca      2536
Leu Thr Trp Asn Val Pro Trp Ala Glu Gly Thr Ile Ser Ala Glu Ala
760                 765                 770                 775

tac gac gag aac aac agg ctg atc ccc gag ggg tcc acc gag ggc aac      2584
Tyr Asp Glu Asn Asn Arg Leu Ile Pro Glu Gly Ser Thr Glu Gly Asn
                780                 785                 790

gcg tcg gtg acc acc acc ggc aag gcc gcg aag ctt aaa gcc gat gcc      2632
Ala Ser Val Thr Thr Thr Gly Lys Ala Ala Lys Leu Lys Ala Asp Ala
                795                 800                 805

gac cgc aag acg atc acc gcg gac ggc aag gac ctg tcg tac atc gag      2680
Asp Arg Lys Thr Ile Thr Ala Asp Gly Lys Asp Leu Ser Tyr Ile Glu
                                        –
```

                    810                          815                          820

gtc gac gtg acc gac gcc aac ggc cat atc gtc ccc gat gcc gcc aac          2728
Val Asp Val Thr Asp Ala Asn Gly His Ile Val Pro Asp Ala Ala Asn
    825                      830                      835

cgc gtc acc ttc gac gtc aag ggc gcc ggc aaa ctg gtc ggc gtc gac          2776
Arg Val Thr Phe Asp Val Lys Gly Ala Gly Lys Leu Val Gly Val Asp
840                  845                  850                      855

aac ggc agc tcg ccg gat cac gac tcc tat cag gcc gac aac cgc aag          2824
Asn Gly Ser Ser Pro Asp His Asp Ser Tyr Gln Ala Asp Asn Arg Lys
                860                  865                      870

gcg ttc agc ggc aag gtg ctc gcc atc gtc cag tcc acc aag gag gcg          2872
Ala Phe Ser Gly Lys Val Leu Ala Ile Val Gln Ser Thr Lys Glu Ala
                875                      880                      885

ggc gag atc acc gtc acc gcc aag gcc gac ggt ctg caa tca tcc aca          2920
Gly Glu Ile Thr Val Thr Ala Lys Ala Asp Gly Leu Gln Ser Ser Thr
                890                      895                      900

gtg aag atc gcc acc acc gcc gtc ccc ggc acc agc acc gag aag acg          2968
Val Lys Ile Ala Thr Thr Ala Val Pro Gly Thr Ser Thr Glu Lys Thr
    905                      910                      915

gtc cgc agc ttc tac tac tcg cgc aac tac tac gtc aag acc ggc aac          3016
Val Arg Ser Phe Tyr Tyr Ser Arg Asn Tyr Tyr Val Lys Thr Gly Asn
920                  925                      930                      935

aag ccg att ctg ccg agt gat gtc gag gtg cgc tac tcc gac ggc acg          3064
Lys Pro Ile Leu Pro Ser Asp Val Glu Val Arg Tyr Ser Asp Gly Thr
                940                      945                      950

tcg gac cgt cag aac gtc aca tgg gac gca gtc agc gac gac cag atc          3112
Ser Asp Arg Gln Asn Val Thr Trp Asp Ala Val Ser Asp Asp Gln Ile
                955                      960                      965

gcc aag gcc ggt tcg ttc agc gtg gcc ggc acg gtc gcc ggg cag aag          3160
Ala Lys Ala Gly Ser Phe Ser Val Ala Gly Thr Val Ala Gly Gln Lys
                970                      975                      980

atc tcc gtg cgc gtg acg atg atc gac gag atc ggt gcg ctg ctc aac          3208
Ile Ser Val Arg Val Thr Met Ile Asp Glu Ile Gly Ala Leu Leu Asn
    985                      990                      995

tat  tcg gcc agc aca ccg  gtc ggc acg ccc gcc  gtg ctg cct ggc          3253

```
Tyr   Ser Ala Ser Thr Pro   Val Gly Thr Pro   Ala Val Leu Pro Gly
1000              1005              1010

tcg cgt ccg gcc gtg ctg   ccc gac ggc acc gtg   acc agc gcg aac        3298
Ser Arg Pro Ala Val Leu   Pro Asp Gly Thr Val   Thr Ser Ala Asn
1015              1020              1025

ttc gcc gtc cac tgg acc   aag ccc gcc gac acc   gtg tac aac acg        3343
Phe Ala Val His Trp Thr   Lys Pro Ala Asp Thr   Val Tyr Asn Thr
1030              1035              1040

gcc ggc acc gtc aag gtc   ccc ggc acc gcc acc   gtc ttc ggc aag        3388
Ala Gly Thr Val Lys Val   Pro Gly Thr Ala Thr   Val Phe Gly Lys
1045              1050              1055

gag ttc aag gtc acc gcg   acg att cgc gtg cag   cgg tcg cag gtc        3433
Glu Phe Lys Val Thr Ala   Thr Ile Arg Val Gln   Arg Ser Gln Val
1060              1065              1070

acc atc ggc agc agc gtc   tcc ggc aat gcg ctg   cgc ctg act cag        3478
Thr Ile Gly Ser Ser Val   Ser Gly Asn Ala Leu   Arg Leu Thr Gln
1075              1080              1085

aac atc ccc gcc gac aag   cag tcc gac acg ctg   gac gcc atc aag        3523
Asn Ile Pro Ala Asp Lys   Gln Ser Asp Thr Leu   Asp Ala Ile Lys
1090              1095              1100

gac ggc tcc acg acc gtc   gac gcc aat acc ggc   ggc ggc gcg aac        3568
Asp Gly Ser Thr Thr Val   Asp Ala Asn Thr Gly   Gly Gly Ala Asn
1105              1110              1115

ccg tca gca tgg acc aac   tgg gcg tac tcg aag   gcc ggc cac aac        3613
Pro Ser Ala Trp Thr Asn   Trp Ala Tyr Ser Lys   Ala Gly His Asn
1120              1125              1130

acc gcc gag atc acc ttc   gag tac gcg acc gag   cag cag ctc ggc        3658
Thr Ala Glu Ile Thr Phe   Glu Tyr Ala Thr Glu   Gln Gln Leu Gly
1135              1140              1145

cag att gtc atg tac ttc   ttc cgc gac agc aac   gcg gtg agg ttc        3703
Gln Ile Val Met Tyr Phe   Phe Arg Asp Ser Asn   Ala Val Arg Phe
1150              1155              1160

ccc gac gcc ggc aag acg   aag atc cag atc tcc   gcg gac ggc aag        3748
Pro Asp Ala Gly Lys Thr   Lys Ile Gln Ile Ser   Ala Asp Gly Lys
1165              1170              1175
```

```
aac  tgg acg gat ctc gct  gcc acg gag acc atc  gcg gcc cag gag        3793
Asn  Trp Thr Asp Leu Ala  Ala Thr Glu Thr Ile  Ala Ala Gln Glu
1180             1185                1190

tcg  tcc gac cga gtc aag  ccg tac acc tat gac  ttc gct ccg gtg        3838
Ser  Ser Asp Arg Val Lys  Pro Tyr Thr Tyr Asp  Phe Ala Pro Val
1195             1200                1205

gga  gcc acg ttc gtc aag  gtc acg gtc acc aac  gcc gac acc aca        3883
Gly  Ala Thr Phe Val Lys  Val Thr Val Thr Asn  Ala Asp Thr Thr
1210             1215                1220

acc  ccc agc ggc gtg gtc  tgc gcc ggc ctg acc  gag atc gag ctg        3928
Thr  Pro Ser Gly Val Val  Cys Ala Gly Leu Thr  Glu Ile Glu Leu
1225             1230                1235

aag  acc gcg acc agc aag  ttc gtc acg aac acg  tcc gcc gcg ctc        3973
Lys  Thr Ala Thr Ser Lys  Phe Val Thr Asn Thr  Ser Ala Ala Leu
1240             1245                1250

tcg  tcg ctg aca gtg aac  ggc acg aag gtc tcc  gac tcc gtg ctc        4018
Ser  Ser Leu Thr Val Asn  Gly Thr Lys Val Ser  Asp Ser Val Leu
1255             1260                1265

gcc  gcc ggc tcc tac aac  acg ccc gcg atc atc  gcg gac gtc aaa        4063
Ala  Ala Gly Ser Tyr Asn  Thr Pro Ala Ile Ile  Ala Asp Val Lys
1270             1275                1280

gcc  gag ggc gaa ggc aac  gcc agc gtc acc gtg  ctg ccc gcg cac        4108
Ala  Glu Gly Glu Gly Asn  Ala Ser Val Thr Val  Leu Pro Ala His
1285             1290                1295

gac  aac gtg atc cgc gtg  atc acc gag tcc gag  gac cac gtc acg        4153
Asp  Asn Val Ile Arg Val  Ile Thr Glu Ser Glu  Asp His Val Thr
1300             1305                1310

cgc  aag acc ttc acc atc  aac ctg ggc acg gag  cag gaa ttc ccc        4198
Arg  Lys Thr Phe Thr Ile  Asn Leu Gly Thr Glu  Gln Glu Phe Pro
1315             1320                1325

gca  gac tcc gat gaa cgc  gac tac ccg gcc gcc  gac atg acg gtc        4243
Ala  Asp Ser Asp Glu Arg  Asp Tyr Pro Ala Ala  Asp Met Thr Val
1330             1335                1340

acc  gtg ggc agc gaa cag  acg tcc ggc acc gcg  acc gaa ggc ccg        4288
Thr  Val Gly Ser Glu Gln  Thr Ser Gly Thr Ala  Thr Glu Gly Pro
1345             1350                1355
```

```
aag  aaa ttc gcg gtc gac   ggc aac acc agc acg   tac tgg cat tcc        4333
Lys  Lys Phe Ala Val Asp   Gly Asn Thr Ser Thr   Tyr Trp His Ser
1360               1365                1370

aac  tgg acg ccc acc acc   gtg aac gac ctg tgg   atc gcc ttc gag        4378
Asn  Trp Thr Pro Thr Thr   Val Asn Asp Leu Trp   Ile Ala Phe Glu
1375               1380                1385

ctc  cag aaa ccc acc aag   ctc gac gcg ctg cgc   tac ctg ccg cgc        4423
Leu  Gln Lys Pro Thr Lys   Leu Asp Ala Leu Arg   Tyr Leu Pro Arg
1390               1395                1400

ccc  gcg ggc agc aag aac   ggc tcc gtc acc gaa   tac aag gtt cag        4468
Pro  Ala Gly Ser Lys Asn   Gly Ser Val Thr Glu   Tyr Lys Val Gln
1405               1410                1415

gtc  agc gat gac ggc acc   aac tgg acc gac gcg   ggc tcc ggc aca        4513
Val  Ser Asp Asp Gly Thr   Asn Trp Thr Asp Ala   Gly Ser Gly Thr
1420               1425                1430

tgg  acc acc gat tac ggc   tgg aag ctc gcc gag   ttc aat cag ccg        4558
Trp  Thr Thr Asp Tyr Gly   Trp Lys Leu Ala Glu   Phe Asn Gln Pro
1435               1440                1445

gtg  acc acc aag cac gtg   cgg ctc aag gcc gtc   cac acc tat gcg        4603
Val  Thr Thr Lys His Val   Arg Leu Lys Ala Val   His Thr Tyr Ala
1450               1455                1460

gat  tcc ggc aac gac aag   ttc atg tcc gcc tcc   gaa atc cgc ctg        4648
Asp  Ser Gly Asn Asp Lys   Phe Met Ser Ala Ser   Glu Ile Arg Leu
1465               1470                1475

cgc  aag gcc gtc gac acc   acc gac atc agc ggc   gcg acc gtg acc        4693
Arg  Lys Ala Val Asp Thr   Thr Asp Ile Ser Gly   Ala Thr Val Thr
1480               1485                1490

gtg  ccc gcc aag ctg acc   gtc gac cgg gtg gac   gcc gac cat ccc        4738
Val  Pro Ala Lys Leu Thr   Val Asp Arg Val Asp   Ala Asp His Pro
1495               1500                1505

gcc  acc ttc gcc acg aag   gac gtg acg gtg acg   ttg ggc gac gcc        4783
Ala  Thr Phe Ala Thr Lys   Asp Val Thr Val Thr   Leu Gly Asp Ala
1510               1515                1520

acg  ctg cgc tac ggc gtg   gac tac ctg ctc gac   tac gcg ggc aac        4828
Thr  Leu Arg Tyr Gly Val   Asp Tyr Leu Leu Asp   Tyr Ala Gly Asn
```

1525                    1530                        1535

```
acc  gcc  gtc  ggc  aag  gcc   acg  gtg  acc  gtg  cgc   ggc  atc  gac  aag        4873
Thr  Ala  Val  Gly  Lys  Ala   Thr  Val  Thr  Val  Arg   Gly  Ile  Asp  Lys
1540                    1545                        1550


tac  tcc  ggc  acc  gtc  gcc   aag  acg  ttc  acc  atc   gaa  ctg  aag  aac        4918
Tyr  Ser  Gly  Thr  Val  Ala   Lys  Thr  Phe  Thr  Ile   Glu  Leu  Lys  Asn
1555                    1560                        1565


gcc  ccg  gcg  ccg  gaa  ccg   acg  ctg  acc  tcg  gtg   agc  gtc  aag  acc        4963
Ala  Pro  Ala  Pro  Glu  Pro   Thr  Leu  Thr  Ser  Val   Ser  Val  Lys  Thr
1570                    1575                        1580


aag  cct  tcc  aag  ctg  acc   tat  gtg  gtc  ggc  gac   gcg  ttc  gac  ccg        5008
Lys  Pro  Ser  Lys  Leu  Thr   Tyr  Val  Val  Gly  Asp   Ala  Phe  Asp  Pro
1585                    1590                        1595


gca  gga  ctg  gtg  ctg  cag   cac  gac  aga  cag  gcc   gat  cgc  ccc  cca        5053
Ala  Gly  Leu  Val  Leu  Gln   His  Asp  Arg  Gln  Ala   Asp  Arg  Pro  Pro
1600                    1605                        1610


cag  cca  ctt  gtt  gga  gaa   cag  gcc  gac  gaa  cgc   gga  ctg  acg  tgc        5098
Gln  Pro  Leu  Val  Gly  Glu   Gln  Ala  Asp  Glu  Arg   Gly  Leu  Thr  Cys
1615                    1620                        1625


gga  acg  cga  tgc  gat  cgc   gtt  gaa  cag  ctg  cgc   aaa  cac  gag  aat        5143
Gly  Thr  Arg  Cys  Asp  Arg   Val  Glu  Gln  Leu  Arg   Lys  His  Glu  Asn
1630                    1635                        1640


cgt  gaa  gcc  cat  cgt  acg   ggc  ctc  gat  cat  ctg   gaa  ttc  gtg  ggt        5188
Arg  Glu  Ala  His  Arg  Thr   Gly  Leu  Asp  His  Leu   Glu  Phe  Val  Gly
1645                    1650                        1655


gcc  gcc  gat  gga  gcg  gtc   ggt  gaa  cag  gcc  acc   ttc  aag  gtg  cat        5233
Ala  Ala  Asp  Gly  Ala  Val   Gly  Glu  Gln  Ala  Thr   Phe  Lys  Val  His
1660                    1665                        1670


gtc  cat  gcc  gat  caa  ggt   gac  ggc  cgc  cat  gat   gat  gcc  gat  gaa        5278
Val  His  Ala  Asp  Gln  Gly   Asp  Gly  Arg  His  Asp   Asp  Ala  Asp  Glu
1675                    1680                        1685


cgc  gat  atc  gat  cca  cat   gtc  cct  gtc  gat  cac   gcg  gtc  ggt  gag        5323
Arg  Asp  Ile  Asp  Pro  His   Val  Pro  Val  Asp  His   Ala  Val  Gly  Glu
1690                    1695                        1700


ctt  gcg  cgg  gct  gcg  tgc   cat  cac  gtc  atc  ggt   ctg  cgg  gtc  gac        5368
```

```
Leu  Ala Arg Ala Ala Cys  His His Val Ile Gly  Leu Arg Val Asp
1705              1710                 1715


acc  cat cgc ctc aag gca  tcc ggc ttc cag atc  ccc gcc gac gac      5413
Thr  His Arg Leu Lys Ala  Ser Gly Phe Gln Ile  Pro Ala Asp Asp
1720             1725                 1730


atg  gcc gag atc gac cgc  atc acc ggc ttc cac  cgc ttc gag cgc      5458
Met  Ala Glu Ile Asp Arg  Ile Thr Gly Phe His  Arg Phe Glu Arg
1735             1740                 1745


cac  gtc ggc tga cgtgattggg cttccccgct gtctggtgcc ggctcgcga        5509
His  Val Gly
1750
```

<210> 2
<211> 1752
<212> PRT
<213> Bifidobacterium bifidum

<400> 2

```
Met Ala Val Arg Arg Leu Gly Gly Arg Ile Val Ala Phe Ala Ala Thr
1               5                   10                  15


Val Ala Leu Ser Ile Pro Leu Gly Leu Leu Thr Asn Ser Ala Trp Ala
        20                  25                  30


Val Glu Asp Ala Thr Arg Ser Asp Ser Thr Thr Gln Met Ser Ser Thr
    35                  40                  45


Pro Glu Val Val Tyr Ser Ser Ala Val Asp Ser Lys Gln Asn Arg Thr
    50                  55                  60


Ser Asp Phe Asp Ala Asn Trp Lys Phe Met Leu Ser Asp Ser Val Gln
65                  70                  75                  80


Ala Gln Asp Pro Ala Phe Asp Asp Ser Ala Trp Gln Gln Val Asp Leu
                85                  90                  95
```

34

```
Pro His Asp Tyr Ser Ile Thr Gln Lys Tyr Ser Gln Ser Asn Glu Ala
        100                 105             110

Glu Ser Ala Tyr Leu Pro Gly Gly Thr Gly Trp Tyr Arg Lys Ser Phe
        115                 120             125

Thr Ile Asp Arg Asp Leu Ala Gly Lys Arg Ile Ala Ile Asn Phe Asp
        130                 135             140

Gly Val Tyr Met Asn Ala Thr Val Trp Phe Asn Gly Val Lys Leu Gly
145                 150             155                 160

Thr His Pro Tyr Gly Tyr Ser Pro Phe Ser Phe Asp Leu Thr Gly Asn
                165             170             175

Ala Lys Phe Gly Gly Glu Asn Thr Ile Val Val Lys Val Glu Asn Arg
                180             185             190

Leu Pro Ser Ser Arg Trp Tyr Ser Gly Ser Gly Ile Tyr Arg Asp Val
        195             200             205

Thr Leu Thr Val Thr Asp Gly Val His Val Gly Asn Asn Gly Val Ala
        210             215             220

Ile Lys Thr Pro Ser Leu Ala Thr Gln Asn Gly Gly Asp Val Thr Met
225             230             235                 240

Asn Leu Thr Thr Lys Val Ala Asn Asp Thr Glu Ala Ala Ala Asn Ile
            245             250             255

Thr Leu Lys Gln Thr Val Phe Pro Lys Gly Gly Lys Thr Asp Ala Ala
        260             265             270

Ile Gly Thr Val Thr Thr Ala Ser Lys Ser Ile Ala Ala Gly Ala Ser
        275             280             285
```

```
Ala Asp Val Thr Ser Thr Ile Thr Ala Ala Ser Pro Lys Leu Trp Ser
    290                 295             300

Ile Lys Asn Pro Asn Leu Tyr Thr Val Arg Thr Glu Val Leu Asn Gly
305                 310             315                 320

Gly Lys Val Leu Asp Thr Tyr Asp Thr Glu Tyr Gly Phe Arg Trp Thr
                325             330                 335

Gly Phe Asp Ala Thr Ser Gly Phe Ser Leu Asn Gly Glu Lys Val Lys
                340             345                 350

Leu Lys Gly Val Ser Met His His Asp Gln Gly Ser Leu Gly Ala Val
                355             360                 365

Ala Asn Arg Arg Ala Ile Glu Arg Gln Val Glu Ile Leu Gln Lys Met
    370                 375             380

Gly Val Asn Ser Ile Arg Thr Thr His Asn Pro Ala Ala Lys Ala Leu
385                 390             395                 400

Ile Asp Val Cys Asn Glu Lys Gly Val Leu Val Val Glu Glu Val Phe
                405             410                 415

Asp Met Trp Asn Arg Ser Lys Asn Gly Asn Thr Glu Asp Tyr Gly Lys
                420             425                 430

Trp Phe Gly Gln Ala Ile Ala Gly Asp Asn Ala Val Leu Gly Gly Asp
                435             440                 445

Lys Asp Glu Thr Trp Ala Lys Phe Asp Leu Thr Ser Thr Ile Asn Arg
                450             455             460

Asp Arg Asn Ala Pro Ser Val Ile Met Trp Ser Leu Gly Asn Glu Met
465                 470             475                 480
```

```
Met Glu Gly Ile Ser Gly Ser Val Ser Gly Phe Pro Ala Thr Ser Ala
            485                 490                 495

Lys Leu Val Ala Trp Thr Lys Ala Ala Asp Ser Thr Arg Pro Met Thr
            500                 505                 510

Tyr Gly Asp Asn Lys Ile Lys Ala Asn Trp Asn Glu Ser Asn Thr Met
            515                 520                 525

Gly Asp Asn Leu Thr Ala Asn Gly Gly Val Val Gly Thr Asn Tyr Ser
            530                 535                 540

Asp Gly Ala Asn Tyr Asp Lys Ile Arg Thr Thr His Pro Ser Trp Ala
545                 550                 555                 560

Ile Tyr Gly Ser Glu Thr Ala Ser Ala Ile Asn Ser Arg Gly Ile Tyr
            565                 570                 575

Asn Arg Thr Thr Gly Gly Ala Gln Ser Ser Asp Lys Gln Leu Thr Ser
            580                 585                 590

Tyr Asp Asn Ser Ala Val Gly Trp Gly Ala Val Ala Ser Ser Ala Trp
            595                 600                 605

Tyr Asp Val Val Gln Arg Asp Phe Val Ala Gly Thr Tyr Val Trp Thr
            610                 615                 620

Gly Phe Asp Tyr Leu Gly Glu Pro Thr Pro Trp Asn Gly Thr Gly Ser
625                 630                 635                 640

Gly Ala Val Gly Ser Leu Ala Val Ala Glu Glu Leu Val Leu Arg His
            645                 650                 655

Arg Arg His Arg Arg Leu Pro Glu Asp Thr Tyr Tyr Phe Tyr Gln Ser
```

```
                    660                 665                 670


Gln Trp Asn Asp Asp Val His Thr Leu His Ile Leu Pro Ala Trp Asn
        675                 680                 685


Glu Asn Val Val Ala Lys Gly Ser Gly Asn Asn Val Pro Val Val Val
        690                 695                 700


Tyr Thr Asp Ala Ala Lys Val Lys Leu Tyr Phe Thr Pro Lys Gly Ser
705                 710                 715                 720


Thr Glu Lys Arg Leu Ile Gly Glu Lys Ser Phe Thr Lys Lys Thr Thr
            725                 730                 735


Ala Ala Gly Tyr Thr Tyr Gln Val Tyr Glu Gly Ser Asp Lys Asp Ser
            740                 745                 750


Thr Ala His Lys Asn Met Tyr Leu Thr Trp Asn Val Pro Trp Ala Glu
            755                 760                 765


Gly Thr Ile Ser Ala Glu Ala Tyr Asp Glu Asn Asn Arg Leu Ile Pro
            770                 775                 780


Glu Gly Ser Thr Glu Gly Asn Ala Ser Val Thr Thr Thr Gly Lys Ala
785                 790                 795                 800


Ala Lys Leu Lys Ala Asp Ala Asp Arg Lys Thr Ile Thr Ala Asp Gly
            805                 810                 815


Lys Asp Leu Ser Tyr Ile Glu Val Asp Val Thr Asp Ala Asn Gly His
            820                 825                 830


Ile Val Pro Asp Ala Ala Asn Arg Val Thr Phe Asp Val Lys Gly Ala
            835                 840                 845
```

```
Gly Lys Leu Val Gly Val Asp Asn Gly Ser Ser Pro Asp His Asp Ser
    850                 855                 860


Tyr Gln Ala Asp Asn Arg Lys Ala Phe Ser Gly Lys Val Leu Ala Ile
865                 870                 875                 880


Val Gln Ser Thr Lys Glu Ala Gly Glu Ile Thr Val Thr Ala Lys Ala
                885                 890                 895


Asp Gly Leu Gln Ser Ser Thr Val Lys Ile Ala Thr Thr Ala Val Pro
            900                 905                 910


Gly Thr Ser Thr Glu Lys Thr Val Arg Ser Phe Tyr Tyr Ser Arg Asn
            915                 920                 925


Tyr Tyr Val Lys Thr Gly Asn Lys Pro Ile Leu Pro Ser Asp Val Glu
    930                 935                 940


Val Arg Tyr Ser Asp Gly Thr Ser Asp Arg Gln Asn Val Thr Trp Asp
945                 950                 955                 960


Ala Val Ser Asp Asp Gln Ile Ala Lys Ala Gly Ser Phe Ser Val Ala
                965                 970                 975


Gly Thr Val Ala Gly Gln Lys Ile Ser Val Arg Val Thr Met Ile Asp
            980                 985                 990


Glu Ile Gly Ala Leu Leu Asn Tyr  Ser Ala Ser Thr Pro  Val Gly Thr
        995                 1000                1005


Pro Ala  Val Leu Pro Gly Ser  Arg Pro Ala Val Leu  Pro Asp Gly
        1010                1015                1020


Thr Val  Thr Ser Ala Asn Phe  Ala Val His Trp Thr  Lys Pro Ala
        1025                1030                1035
```

```
Asp Thr  Val Tyr Asn Thr Ala  Gly Thr Val Lys Val  Pro Gly Thr
    1040             1045              1050

Ala Thr  Val Phe Gly Lys Glu  Phe Lys Val Thr Ala  Thr Ile Arg
    1055             1060              1065

Val Gln  Arg Ser Gln Val Thr  Ile Gly Ser Ser Val  Ser Gly Asn
    1070             1075              1080

Ala Leu  Arg Leu Thr Gln Asn  Ile Pro Ala Asp Lys  Gln Ser Asp
    1085             1090              1095

Thr Leu  Asp Ala Ile Lys Asp  Gly Ser Thr Thr Val  Asp Ala Asn
    1100             1105              1110

Thr Gly  Gly Gly Ala Asn Pro  Ser Ala Trp Thr Asn  Trp Ala Tyr
    1115             1120              1125

Ser Lys  Ala Gly His Asn Thr  Ala Glu Ile Thr Phe  Glu Tyr Ala
    1130             1135              1140

Thr Glu  Gln Gln Leu Gly Gln  Ile Val Met Tyr Phe  Phe Arg Asp
    1145             1150              1155

Ser Asn  Ala Val Arg Phe Pro  Asp Ala Gly Lys Thr  Lys Ile Gln
    1160             1165              1170

Ile Ser  Ala Asp Gly Lys Asn  Trp Thr Asp Leu Ala  Ala Thr Glu
    1175             1180              1185

Thr Ile  Ala Ala Gln Glu Ser  Ser Asp Arg Val Lys  Pro Tyr Thr
    1190             1195              1200

Tyr Asp  Phe Ala Pro Val Gly  Ala Thr Phe Val Lys  Val Thr Val
    1205             1210              1215
```

```
Thr Asn  Ala Asp Thr Thr Thr  Pro Ser Gly Val Val  Cys Ala Gly
    1220             1225          1230

Leu Thr  Glu Ile Glu Leu Lys  Thr Ala Thr Ser Lys  Phe Val Thr
    1235             1240          1245

Asn Thr  Ser Ala Ala Leu Ser  Ser Leu Thr Val Asn  Gly Thr Lys
    1250             1255          1260

Val Ser  Asp Ser Val Leu Ala  Ala Gly Ser Tyr Asn  Thr Pro Ala
    1265             1270          1275

Ile Ile  Ala Asp Val Lys Ala  Glu Gly Glu Gly Asn  Ala Ser Val
    1280             1285          1290

Thr Val  Leu Pro Ala His Asp  Asn Val Ile Arg Val  Ile Thr Glu
    1295             1300          1305

Ser Glu  Asp His Val Thr Arg  Lys Thr Phe Thr Ile  Asn Leu Gly
    1310             1315          1320

Thr Glu  Gln Glu Phe Pro Ala  Asp Ser Asp Glu Arg  Asp Tyr Pro
    1325             1330          1335

Ala Ala  Asp Met Thr Val Thr  Val Gly Ser Glu Gln  Thr Ser Gly
    1340             1345          1350

Thr Ala  Thr Glu Gly Pro Lys  Lys Phe Ala Val Asp  Gly Asn Thr
    1355             1360          1365

Ser Thr  Tyr Trp His Ser Asn  Trp Thr Pro Thr Thr  Val Asn Asp
    1370             1375          1380

Leu Trp  Ile Ala Phe Glu Leu  Gln Lys Pro Thr Lys  Leu Asp Ala
```

```
                    1385                    1390                    1395


        Leu Arg  Tyr Leu Pro Arg Pro  Ala Gly Ser Lys Asn  Gly Ser Val
            1400                    1405                    1410


        Thr Glu  Tyr Lys Val Gln Val  Ser Asp Asp Gly Thr  Asn Trp Thr
            1415                    1420                    1425


        Asp Ala  Gly Ser Gly Thr Trp  Thr Thr Asp Tyr Gly  Trp Lys Leu
            1430                    1435                    1440


        Ala Glu  Phe Asn Gln Pro Val  Thr Thr Lys His Val  Arg Leu Lys
            1445                    1450                    1455


        Ala Val  His Thr Tyr Ala Asp  Ser Gly Asn Asp Lys  Phe Met Ser
            1460                    1465                    1470


        Ala Ser  Glu Ile Arg Leu Arg  Lys Ala Val Asp Thr  Thr Asp Ile
            1475                    1480                    1485


        Ser Gly  Ala Thr Val Thr Val  Pro Ala Lys Leu Thr  Val Asp Arg
            1490                    1495                    1500


        Val Asp  Ala Asp His Pro Ala  Thr Phe Ala Thr Lys  Asp Val Thr
            1505                    1510                    1515


        Val Thr  Leu Gly Asp Ala Thr  Leu Arg Tyr Gly Val  Asp Tyr Leu
            1520                    1525                    1530


        Leu Asp  Tyr Ala Gly Asn Thr  Ala Val Gly Lys Ala  Thr Val Thr
            1535                    1540                    1545


        Val Arg  Gly Ile Asp Lys Tyr  Ser Gly Thr Val Ala  Lys Thr Phe
            1550                    1555                    1560
```

Thr Ile  Glu Leu Lys Asn Ala  Pro Ala Pro Glu Pro  Thr Leu Thr
    1565              1570              1575

Ser Val  Ser Val Lys Thr Lys  Pro Ser Lys Leu Thr  Tyr Val Val
    1580              1585              1590

Gly Asp  Ala Phe Asp Pro Ala  Gly Leu Val Leu Gln  His Asp Arg
    1595              1600              1605

Gln Ala  Asp Arg Pro Pro Gln  Pro Leu Val Gly Glu  Gln Ala Asp
    1610              1615              1620

Glu Arg  Gly Leu Thr Cys Gly  Thr Arg Cys Asp Arg  Val Glu Gln
    1625              1630              1635

Leu Arg  Lys His Glu Asn Arg  Glu Ala His Arg Thr  Gly Leu Asp
    1640              1645              1650

His Leu  Glu Phe Val Gly Ala  Ala Asp Gly Ala Val  Gly Glu Gln
    1655              1660              1665

Ala Thr  Phe Lys Val His Val  His Ala Asp Gln Gly  Asp Gly Arg
    1670              1675              1680

His Asp  Asp Ala Asp Glu Arg  Asp Ile Asp Pro His  Val Pro Val
    1685              1690              1695

Asp His  Ala Val Gly Glu Leu  Ala Arg Ala Ala Cys  His His Val
    1700              1705              1710

Ile Gly  Leu Arg Val Asp Thr  His Arg Leu Lys Ala  Ser Gly Phe
    1715              1720              1725

Gln Ile  Pro Ala Asp Asp Met  Ala Glu Ile Asp Arg  Ile Thr Gly
    1730              1735              1740

        Phe His  Arg Phe Glu Arg His  Val Gly
            1745              1750